# EUROPEAN PATENT SPECIFICATION

(11) **EP 4 400 046 B1**
(45) Date of publication and mention of the grant of the patent: **09.09.2026**
(21) Application number: 23202328.3
(22) Date of filing: 09.10.2023
(51) Int. Cl.: G16H 40/40, G16H 40/63, G16H 40/67, A61B 5/145, A61B 5/1486, A61B 5/1495, A61B 5/00, A61M 5/172, G16H 20/17

(54) **SYSTEMS AND METHODS FOR DETECTING PRESENCE OF EXCIPIENT OF INSULIN**
SYSTEME UND VERFAHREN ZUM NACHWEIS DES VORHANDENSEINS EINES HILFSSTOFFS VON INSULIN
SYSTÈMES ET PROCÉDÉS DE DÉTECTION DE LA PRÉSENCE D'EXCIPIENT D'INSULINE

(30) Priority: 14.10.2022 US 202263416005 P; 20.09.2023 US 202318470633
(43) Date of publication of application: 17.07.2024
(73) Proprietor: Medtronic MiniMed, Inc., Northridge, CA 91325 (US)
(72) Inventor: Miller, Margaret R., Northridge, 91326 (US); Garai, Ellis, Northridge, 91325 (US)
(74) Representative: Zimmermann & Partner Patentanwälte mbB

(56) References cited:
- US-A1- 2013 328 573
- US-A1- 2021 386 331

## Description

### FIELD

The present disclosure relates generally to glucose sensor technology for sensing a variety of physiological parameters, e.g., glucose concentration, and more particularly, to glucose sensor systems and methods for detecting presence of an excipient of insulin.

### BACKGROUND

Continuous glucose monitoring (CGM) has been used to continuously monitor glucose levels in a user and alerts the user when the glucose level is outside of the normal range. The glucose levels can be measured based on raw glucose sensor values (e.g., the sensor current) and the counter voltage. When the glucose level is greater than a safe range, insulin is injected into the user. Generally, one or more excipients in the insulin are also injected to provide stability and antimicrobial properties. The excipients, however, may affect the sensor signals, thereby affecting the glucose levels.
US 2021/0386331 A1 relates to methods, systems and devices for improved sensors for continuous glucose monitoring. US 2013/0328573 Al relates to an application of electrochemical impedance spectroscopy in sensor systems, devices and related methods.

### SUMMARY

The present disclosure relates to systems and methods for detecting the presence of an excipient in insulin so that a proper glucose level can be provided. When insulin is injected, excipients of the insulin are also injected and cause changes in signals measured by a glucose sensor. Thus, by monitoring changes in the signals, the presence of excipients may be detected. In accordance with aspects of the present disclosure, a glucose sensor includes a working electrode configured to provide a current signal (Isig_{WE1}) based on a level of glucose, a background electrode configured to provide a current signal (Isig_{WE2}) based on presence of an excipient of insulin, and a controller. The controller is configured to monitor the Isig_{WE1} at the working electrode, monitor the Isig_{WE2} at the background electrode, monitor at least one electrochemical impedance spectroscopy (EIS) parameter at the working electrode, calculate a change in the at least one EIS parameter after an injection of insulin, and detect the presence of an excipient of insulin based on the change. In a case where the presence of the excipient of insulin is detected, the controller is further configured to compensate the Isig_{WE1} based on a predetermined relationship between the Isig_{WE1} and the Isig_{WE2}, and output the compensated IsigWE1 (Isig_{COMP}).

In various aspects, the at least one EIS parameter is a real impedance at 0.1 Hz.

In various aspects, the at least one EIS parameter is an imaginary impedance at 0.1 Hz.

In various aspects, the predetermined relationship between Isig_{WE1} and the Isig_{WE2} is a linear relationship. The Isig_{COMP} is equal to: Isig_{WE1} - α * Isig_{WE2}, where α is a slope of the linear relationship.

In various aspects, the slope has a profile based on a number of injections of insulin. The glucose sensor further includes a memory storing the number of injections of insulin and the profile.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a glucose sensor includes a cannula connected to an insulin pump and configured to inject insulin therethrough, a working electrode configured to provide a current signal (Isig_{WE1}) based on a level of glucose, a background electrode configured to provide a current signal (Isig_{WE2}) based on presence of excipient of insulin, and a controller. The controller is configured to monitor the IsigWE1 at the working electrode, monitor the Isig_{WE2} at the background electrode, monitor at least one electrochemical impedance spectroscopy (EIS) parameter at the working electrode, calculate a change in the at least one EIS parameter after an injection of insulin, and detect the presence of an excipient of insulin based on the change. In a case where the presence of the excipient of insulin is detected, the controller is further configured to compensate the Isig_{WE1} based on a predetermined relationship between the Isig_{WE1} and the Isig_{WE2}, and output the compensated Isig_{WE1} (Isig_{COMP}).

In various aspects, the at least one EIS parameter is a real impedance at 0.1 Hz.

In various aspects, the at least one EIS parameter is an imaginary impedance at 0.1 Hz.

In various aspects, the predetermined relationship between Isig_{WE1} and the Isig_{WE2} is a linear relationship. The Isig_{COMP} is equal to: Isig_{WE1} - α * Isig_{WE2}, where α is a slope of the linear relationship.

In various aspects, the slope has a profile based on a number of injections of insulin. The glucose sensor further includes a memory storing the number of inj ections of insulin and the profile.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a method for compensating a level of glucose from a glucose sensor based on the presence of an excipient of insulin includes monitoring a current signal (Isig_{WE1}) at a working electrode of the glucose sensor, monitoring a current signal (Isig_{WE2}) at a background electrode of the glucose sensor, monitoring at least one electrochemical impedance spectroscopy (EIS) parameter at the working electrode, calculating a change in the at least one EIS parameter after an injection of insulin, and detecting presence of an excipient of insulin based on the change. In a case where the presence of the excipient of insulin is detected, the method further includes compensating the Isig_{WE1} based on a predetermined relationship between the Isig_{WE1} and the Isig_{WE2}, and outputting the compensated Isig_{WE1} (Isig_{COMP}).

In various aspects, the at least one EIS parameter is a real impedance at 0.1 Hz.

In various aspects, the at least one EIS parameter is an imaginary impedance at 0.1 Hz.

In various aspects, the predetermined relationship between Isig_{WE1} and the Isig_{WE2} is a linear relationship. The Isig_{COMP} is equal to: Isig_{WE1} - α * Isig_{WE2}, where α is a slope of the linear relationship.

In various aspects, the slope has a profile based on a number of injections of insulin.

In various aspect, the method further includes storing the number of injections of insulin and the profile in a memory of the glucose sensor.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a nontransitory computer-readable medium stores instructions that, when executed by a computing device, cause the computing device to perform a method for compensating a level of glucose from a glucose sensor based on presence of an excipient of insulin. The includes monitoring a current signal (Isig_{WE1}) at a working electrode of the glucose sensor, monitoring a current signal (Isig_{WE2}) at a background electrode of the glucose sensor, monitoring at least one electrochemical impedance spectroscopy (EIS) parameter at the working electrode, calculating a change in the at least one EIS parameter after an injection of insulin, and detecting the presence of an excipient of insulin based on the change. In a case where the presence of the excipient of insulin is detected, the method further includes compensating the Isig_{WE1} based on a predetermined relationship between the Isig_{WE1} and the Isig_{WE2}, and outputting the compensated Isig_{WE1} (Isig_{COMP}).

In accordance with aspects of the present disclosure, a glucose sensor includes a working electrode configured to provide a current signal (Isig_{WE1}) based on a level of glucose, a background electrode configured to provide a current signal (Isig_{WE2}) based on presence of excipient of insulin, and a controller. The controller is configured to monitor a current signal (IsigWE1) at the working electrode, monitor a current signal (IsigWE2) at the background electrode, calculate a change in the IsigWE2 after an injection of insulin, and detect the presence of an excipient of insulin based on the change. In a case where the presence of the excipient of insulin is detected, the controller is further configured to compensate the Isig_{WE1} based on a predetermined relationship between the Isig_{WE1} and the Isig_{WE2}, and output the compensated Isig_{WE1} (Isig_{COMP}).

In various aspects, the predetermined relationship between the Isig_{WE1} and the Isig_{WE2} is a linear relationship. The Isig_{COMP} is equal to: Isig_{WE1} - α * Isig_{WE2}, where α is a slope of the linear relationship.

In various aspects, the slope has a profile based on a number of injections of insulin.

In various aspects, the glucose sensor further includes a memory storing the number of injections of insulin and the profile.

In various aspects, a range of voltage applied to the background electrode is from 500 mV to 600 mV.

In various aspects, the controller is further configured to monitor an electrochemical impedance spectroscopy (EIS) signal at the working electrode, and confirm the presence of the excipient of insulin based on the EIS signal of at least one parameter.

In various aspects, the at least one parameter is a real impedance at 0.1 Hz of the EIS signal.

In various aspects, the at least one parameter is an imaginary impedance at 0.1 Hz of the EIS signal.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a glucose sensor system includes a cannula connected to an insulin pump and configured to inject insulin therethrough, a working electrode configured to provide a current signal (Isig_{WE1}) based on a level of glucose, a background electrode configured to provide a current signal (Isig_{WE2}) based on the presence of an excipient of insulin, and a controller. The controller is configured to monitor the Isig_{WE1} at the working electrode, monitor the Isig_{WE2} at the background electrode, calculate a change in the Isig_{WE2} after an injection of insulin, and detect presence of an excipient of insulin based on the change. In a case where the presence of the excipient of insulin is detected, the controller is further configured to compensate the Isig_{WE1} based on a predetermined relationship between the Isig_{WE1} and the Isig_{WE2}, and output the compensated Isig_{WE1} (Isig_{COMP}).

In various aspects, the predetermined relationship between the Isig_{WE1} and the Isig_{WE2} is a linear relationship. The Isig_{COMP} is equal to: Isig_{WE1} - α * Isig_{WE2}, where α is a slope of the linear relationship.

In various aspects, the slope depends on a distance between the cannula and the working electrode.

In various aspects, the slope has a profile based on a number of injections of insulin. The profile is based on a distance between the cannula and the background electrode. The glucose sensor system further includes a memory storing the number of injections of insulin and the profile.

In various aspects, a range of voltage applied to the background electrode is from 500 mV to 600 mV.

In various aspects, the controller is further configured to monitor at least one electrochemical impedance spectroscopy (EIS) parameter at the working electrode, and confirm the presence of the excipient of insulin based on the at least one EIS parameter. The at least one EIS parameter is a real impedance at 0.1 Hz of the EIS signal. The at least one EIS parameter is an imaginary impedance at 0.1 Hz of the EIS signal.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a method for compensating a level of glucose from a glucose sensor based on the presence of an excipient of insulin includes monitoring a current signal (IsigWE1) at a working electrode of the glucose sensor, monitoring a current signal (IsigWE2) at a background electrode of the glucose sensor, calculating a change in the IsigWE2 after an injection of insulin, and detecting the presence of an excipient of insulin based on the change. In a case where the presence of the excipient of insulin is detected, the method further includes compensating the Isig_{WE1} based on a predetermined relationship between the Isig_{WE1} and the Isig_{WE2}, and outputting the compensated Isig_{WE1} (Isig_{COMP}).

In various aspects, the predetermined relationship between the Isig_{WE1} and the Isig_{WE2} is a linear relationship. The Isig_{COMP} is equal to Isig_{WE1} - α * Isig_{WE2}, where α is a slope of the linear relationship.

In various aspects, the slope depends on a distance between a cannula, through which insulin is injected, and the working electrode.

In various aspects, the slope has a profile based on a number of injections of insulin. The more time insulin is injected, the lower the slope is.

In various aspects, a range of voltage applied to the background electrode is from 500 mV to 600 mV.

In various aspects, the method further includes monitoring at least one electrochemical impedance spectroscopy (EIS) parameter at the working electrode, and confirming the presence of the excipient of insulin based on the at least one EIS parameter. The at least one EIS parameter is a real impedance at 0.1 Hz or the at least one EIS parameter is an imaginary impedance at 0.1 Hz.

In various aspects, the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

In accordance with aspects of the present disclosure, a nontransitory computer-readable medium stores instructions that, when executed by a computing device, cause the computing device to perform a method for compensating a level of glucose from a glucose sensor based on the presence of an excipient of insulin. The method includes monitoring a current signal (IsigWE1) at a working electrode of the glucose sensor, monitoring a current signal (IsigWE2) at a background electrode of the glucose sensor, calculating a change in the IsigWE2 after an injection of insulin, and detecting presence of an excipient of insulin based on the change. In a case where the presence of the excipient of insulin is detected, the method further includes compensating the Isig_{WE1} based on a predetermined relationship between the Isig_{WE1} and the Isig_{WE2}, and outputting the compensated Isig_{WE1} (Isig_{COMP}).

Further disclosed herein is a glucose sensor that includes a working electrode configured to provide a current signal (Isig_{WE1}) based on a level of glucose, a background electrode configured to provide a current signal (Isig_{WE2}) based on a presence of an excipient of insulin, and a controller. The controller is configured to monitor the Isig_{WE1} at the working electrode, monitor the Isig_{WE2} at the background electrode, monitor at least one electrochemical impedance spectroscopy (EIS) parameter at the working electrode, and calculate a change in the at least one EIS parameter, detect presence of the excipient of insulin based on the change. In a case where the presence of the excipient of insulin is detected, the controller is further configured to compensate the Isig_{WE1} based on a predetermined relationship between the Isig_{WE1} and the Isig_{WE2}, and output the compensated Isig_{WE1} (Isig_{COMP}).

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A detailed description of aspects of the disclosure will be made with reference to the accompanying drawings, wherein like numerals designate corresponding parts in the figures.
FIG. 1 illustrates a perspective view of a subcutaneous sensor insertion set and a block diagram of a sensor electronics device in accordance with one or more aspects.
FIG. 2 illustrates a substrate having two sides, a first side which contains an electrode configuration and a second side which contains electronic circuitry in accordance with one or more aspects.
FIG. 3 illustrates a block diagram of an electronic circuit for sensing an output of a sensor in accordance with one or more aspects.
FIG. 4 illustrates a block diagram of a sensor electronics device and a sensor including a plurality of electrodes in accordance with one or more aspects.
FIG. 5 illustrates an alternative aspect including a sensor and a sensor electronics device in accordance with one or more aspects.
FIG. 6 illustrates an electronic block diagram of the sensor electrodes and a voltage being applied to the sensor electrodes in accordance with one or more aspects.
FIG. 7 illustrates a block diagram of a glucose sensor system in accordance with one or more aspects.
FIG. 8 illustrates a graphical representation of false increases in a current signal (Isig) at a working electrode of a glucose sensor due to presence of excipients in insulin.
FIG. 9 illustrates graphical representations of changes in Isig and voltage signals due to presence of excipients in insulin in accordance with one or more aspects.
FIG. 10 illustrates graphical representations of changes in real and impedance in EIS signal at a working electrode in accordance with the one or more aspects.
FIG. 11 illustrates graphical representations of changes in real and impedance in EIS signal at a background electrode in accordance with the one or more aspects.
FIG. 12 illustrates graphical representations of changes in Isig and voltage signals due to presence of excipients in insulin in accordance with one or more aspects.
FIG. 13 illustrates graphical representations of changes in real and impedance in EIS signal at a working electrode in accordance with the one or more aspects.
FIG. 14 illustrates graphical representations of changes in real and impedance in EIS signal at a background electrode in accordance with the one or more aspects.
FIG. 15 illustrates graphical representations of changes in Isig at the working electrode and EIS signals at the background electrode due to presence of excipients in insulin and application of voltage to the working electrode in accordance with one or more aspects.
FIG. 16 illustrates a graphical illustration of a linear relationship between amplitudes of the Isig at the working electrode and concentrations of insulin at the start of the first insulin injection in accordance with one or more aspects.
FIG. 17 illustrates a graphical illustration of a linear relationship between amplitudes of the Isig at the working electrode and concentrations of insulin at the end of the first insulin injection in accordance with one or more aspects.
FIG. 18 illustrates a graphical illustration of a linear relationship between amplitudes of the Isig at the working electrode and concentrations of insulin at the start of the second insulin injection in accordance with one or more aspects.
FIG. 19 illustrates a flowchart of a method for detecting presence of an excipient in insulin in accordance with one or more aspects.
FIG. 20 illustrates a flowchart of a method for detecting presence of an excipient in insulin in accordance with one or more aspects.

### DETAILED DESCRIPTION

In the following description, reference is made to the accompanying drawings which form a part hereof and which illustrate several aspects of the present disclosure. It is understood that other aspects may be utilized, and structural and operational changes may be made without departing from the scope of the present disclosure.

The aspects herein are described below with reference to flowchart illustrations of methods, systems, devices, apparatus, and programming and computer program products. It will be understood that each block of the flowchart illustrations, and combinations of blocks in the flowchart illustrations, can be implemented by programming instructions, including computer program instructions (as can any menu screens described in the figures). These computer program instructions may be loaded onto a computer or other programmable data processing apparatus (such as a controller, microcontroller, or processor in a sensor electronics device) to produce a machine, such that the instructions which execute on the computer or other programmable data processing apparatus create instructions for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks, and/or menus presented herein. Programming instructions may also be stored in and/or implemented via electronic circuitry, including integrated circuits (ICs) and Application Specific Integrated Circuits (ASICs) used in conjunction with sensor devices, apparatuses, and systems.

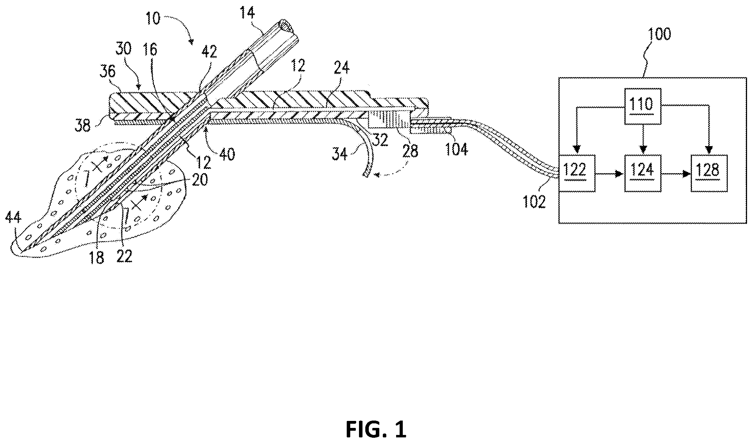

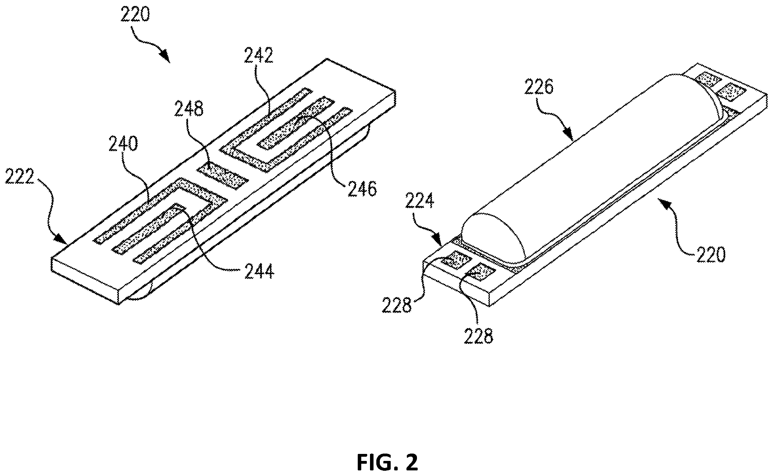

FIG. 1 is a perspective view of a subcutaneous sensor insertion set and a block diagram of a sensor electronics device according to various aspects of the disclosure. As illustrated in FIG. 1, a subcutaneous sensor set 10 is provided for subcutaneous placement of an active portion of a flexible sensor 12 (see, e.g., FIG. 2), or the like, at a selected site in the body of a user. The subcutaneous or percutaneous portion of the sensor set 10 includes a hollow, slotted insertion needle 14, and a cannula 16. The needle 14 is used to facilitate quick and easy subcutaneous placement of the cannula 16 at the subcutaneous insertion site. Inside the cannula 16 is a sensing portion 18 of the sensor 12 to expose one or more sensor electrodes 20 to the user's bodily fluids through a window 22 formed in the cannula 16. In an aspect of the disclosure, the one or more sensor electrodes 20 may include a counter electrode, a reference electrode, and one or more working electrodes. After insertion, the insertion needle 14 is withdrawn to leave the cannula 16 with the sensing portion 18 and the sensor electrodes 20 in place at the selected insertion site.

In particular aspects, the subcutaneous sensor set 10 facilitates accurate placement of a flexible thin film electrochemical sensor 12 of the type used for monitoring specific blood parameters representative of a user's condition. The sensor 12 monitors glucose levels in the body and may be used in conjunction with automated or semi-automated medication infusion pumps of the external or implantable type as described, e.g., in U.S. Patent Nos. 4,562,751; 4,678,408; 4,685,903 or 4,573,994, to control delivery of insulin to a diabetic patient.

Particular aspects of the flexible electrochemical sensor 12 are constructed in accordance with thin film mask techniques to include elongated thin film conductors embedded or encased between layers of a selected insulative material such as polyimide film or sheet, and membranes. The sensor electrodes 20 at a tip end of the sensing portion 18 are exposed through one of the insulative layers for direct contact with patient blood or other body fluids, when the sensing portion 18 (or active portion) of the sensor 12 is subcutaneously placed at an insertion site. The sensing portion 18 is joined to a connection portion 24 that terminates in conductive contact pads, or the like, which are also exposed through one of the insulative layers. In alternative aspects, other types of implantable sensors, such as chemical based, optical based, or the like, may be used.

As is known in the art, the connection portion 24 and the contact pads are generally adapted for a direct wired electrical connection to a suitable monitor or sensor electronics device 100 for monitoring a user's condition in response to signals derived from the sensor electrodes 20. Further description of flexible thin film sensors of this general type may be found, e.g., in U.S. Patent No. 5,391,250. The connection portion 24 may be conveniently connected electrically to the monitor or sensor electronics device 100 or by a connector block 28 (or the like) as shown and described, e.g., in U.S. Patent No. 5,482,473. Thus, in accordance with aspects of the present disclosure, subcutaneous sensor sets 10 may be configured or formed to work with either a wired or a wireless characteristic monitor system.

The sensor electrodes 20 may be used in a variety of sensing applications and may be configured in a variety of ways. For example, the sensor electrodes 20 may be used in physiological parameter sensing applications in which some type of biomolecule is used as a catalytic agent. For example, the sensor electrodes 20 may be used in a glucose and oxygen sensor having a glucose oxidase (GOx) enzyme catalyzing a reaction with the sensor electrodes 20. The reaction produces Gluconic Acid (C6H12O7) and Hydrogen Peroxide (H2O2) in proportion to the amount of glucose present.

The sensor electrodes 20, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

The monitor 100 may also be referred to as a sensor electronics device 100. The monitor 100 may include a power source 110, a sensor interface 122, processing electronics 124, and data formatting electronics 128. The monitor 100 may be coupled to the sensor set 10 by a cable 102 through a connector that is electrically coupled to the connector block 28 of the connection portion 24. In an alternative aspect, the cable 102 may be omitted. In this aspect of the disclosure, the monitor 100 may include an appropriate connector for direct connection to the connection portion 104 of the sensor set 10. The sensor set 10 may be modified to have the connector portion 104 positioned at a different location, e.g., on top of the sensor set 10 to facilitate placement of the monitor 100 over the sensor set 10.

In aspects of the disclosure, the sensor interface 122, the processing electronics 124, and the data formatting electronics 128 are formed as separate semiconductor chips, however, alternative aspects may combine the various semiconductor chips into a single or multiple customized semiconductor chips. The sensor interface 122 connects with the cable 102 that is connected with the sensor set 10.

The power source 110 may be a battery. The battery can include three series silver oxide battery cells. In alternative aspects, different battery chemistries may be utilized, such as lithium based chemistries, alkaline batteries, nickel metalhydride, or the like, and a different number of batteries may be used. The monitor 100 provides power to the sensor set via the power source 110, through the cable 102 and cable connector 104. In an aspect of the disclosure, the power is a voltage provided to the sensor set 10. In an aspect of the disclosure, the power is a current provided to the sensor set 10. In an aspect of the disclosure, the power is a voltage provided at a specific voltage to the sensor set 10.

FIG. 2 illustrates an implantable sensor and electronics for driving the implantable sensor according to an aspect of the present disclosure. FIG. 2 shows a substrate or flex220 having two sides; a first side 222 which contains an electrode configuration and a second side 224 of which contains electronic circuitry. As in FIG. 2, the first side 222 of the substrate includes two counter electrode-working electrode pairs 240, 242, 244, 246 on opposite sides of a reference electrode 248. A second side 224 of the substrate includes electronic circuitry. As shown, the electronic circuitry may be enclosed in a hermetically sealed casing 226, providing a protective housing for the electronic circuitry. This allows the sensor substrate 220 to be inserted into a vascular environment or other environment which may subject the electronic circuitry to fluids. By sealing the electronic circuitry in a hermetically sealed casing 226, the electronic circuitry may operate without risk of short circuiting by the surrounding fluids. Also shown in FIG. 2, pads 228 are connected to the input and output lines of the electronic circuitry. The electronic circuitry itself may be fabricated in a variety of ways. According to an aspect of the present disclosure, the electronic circuitry may be fabricated as an integrated circuit using techniques common in the industry.

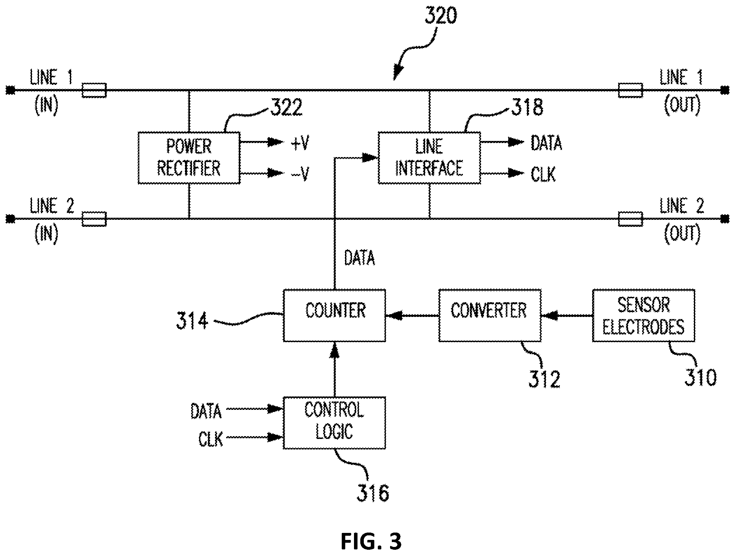

FIG. 3 illustrates a general block diagram of an electronic circuit for sensing an output of a sensor according to aspects of the present disclosure. At least one pair of sensor electrodes 310 may interface to a data converter 312, the output of which may interface to a counter 314. The counter 314 may be controlled by control logic 316. The output of the counter 314 may connect to a line interface 318. The line interface 318 may be connected to input and output lines 320 and may also connect to the control logic 316. The input and output lines 320 may also be connected to a power rectifier 322.

The sensor electrodes 310 may be used in a variety of sensing applications and may be configured in a variety of ways. For example, the sensor electrodes 310 may be used in physiological parameter sensing applications in which some type of biomolecule is used as a catalytic agent. For example, the sensor electrodes 310 may be used in a glucose and oxygen sensor having a glucose oxidase (GOx) enzyme catalyzing a reaction with the sensor electrodes 310. The sensor electrodes 310, along with a biomolecule or some other catalytic agent, may be placed in a human body in a vascular or non-vascular environment. For example, the sensor electrodes 310 and biomolecule may be placed in a vein and be subjected to a blood stream.

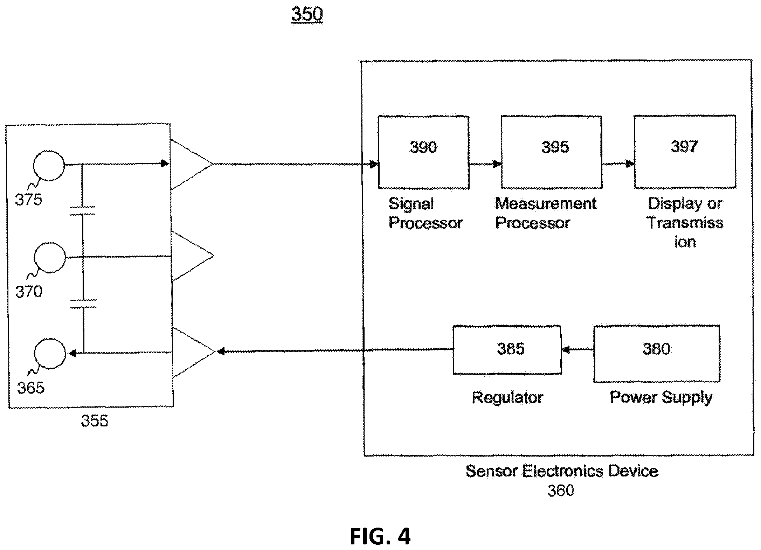

FIG. 4 illustrates a block diagram of a sensor electronics device and a sensor including a plurality of electrodes according to an aspect of the disclosure. The sensor set or system 350 includes a sensor 355 and a sensor electronics device 360. The sensor 355 includes a counter electrode 365, a reference electrode 370, and a working electrode 375. The sensor electronics device 360 includes a power supply 380, a regulator 385, a signal processor 390, a measurement processor 395, and a display/transmission module 397. The power supply 380 provides power (in the form of either a voltage, a current, or a voltage including a current) to the regulator 385. The regulator 385 transmits a regulated voltage to the sensor 355. In an aspect of the disclosure, the regulator 385 transmits a voltage to the counter electrode 365 of the sensor 355.

The sensor 355 creates a sensor signal indicative of a concentration of a physiological characteristic being measured. For example, the sensor signal may be indicative of a blood glucose reading. In an aspect of the disclosure, utilizing subcutaneous sensors, the sensor signal may represent a level of hydrogen peroxide in a subject. In an aspect of the disclosure, where blood or cranial sensors are utilized, the amount of oxygen is being measured by the sensor and is represented by the sensor signal. In an aspect of the disclosure, utilizing implantable or long-term sensors, the sensor signal may represent a level of oxygen in the subject. The sensor signal may be measured at the working electrode 375. In an aspect of the disclosure, the sensor signal may be a current measured at the working electrode. In an aspect of the disclosure, the sensor signal may be a voltage measured at the working electrode.

The signal processor 390 receives the sensor signal (e.g., a measured current or voltage) after the sensor signal is measured at the sensor 355 (e.g., the working electrode). The signal processor 390 processes the sensor signal and generates a processed sensor signal. The measurement processor 395 receives the processed sensor signal and calibrates the processed sensor signal utilizing reference values. In an aspect of the disclosure, the reference values are stored in a reference memory and provided to the measurement processor 395. The measurement processor 395 generates sensor measurements. The sensor measurements may be stored in a measurement memory (not shown). The sensor measurements may be sent to a display/transmission device to be either displayed on a display in a housing with the sensor electronics or transmitted to an external device.

The sensor electronics device 360 may be a monitor which includes a display to display physiological characteristics readings. The sensor electronics device 360 may also be installed in a desktop computer, a pager, a television including communications capabilities, a laptop computer, a server, a network computer, a personal digital assistant (PDA), a portable telephone including computer functions, an infusion pump including a display, a glucose sensor including a display, and/or a combination infusion pump/glucose sensor. The sensor electronics device 360 may be housed in a cellular phone, a smartphone, a network device, a home network device, and/or other appliance connected to a home network.

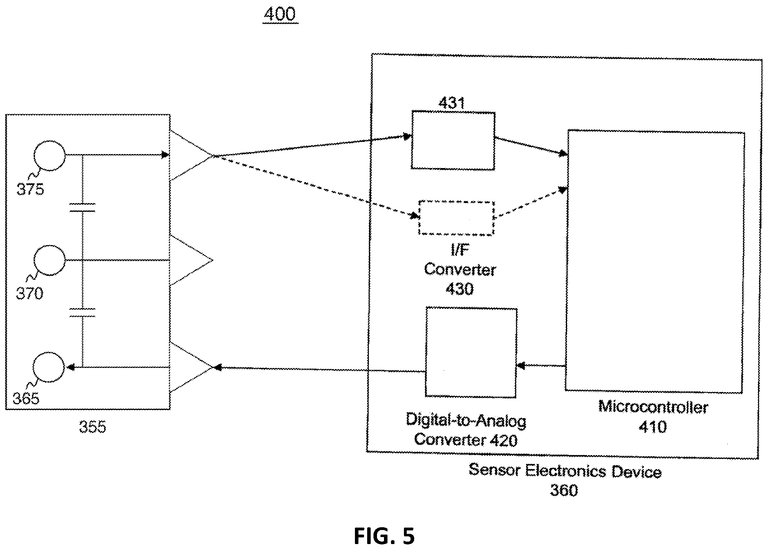

FIG. 5 illustrates an alternative aspect including a sensor and a sensor electronics device according to an aspect of the present disclosure. The sensor set or sensor system 400 includes a sensor electronics device 360 and a sensor 355. The sensor 355 includes a counter electrode 365, a reference electrode 370, and a working electrode 375. The sensor electronics device 360 includes a microcontroller 410 and a digital-to-analog converter (DAC) 420. The sensor electronics device 360 may also include a current-to-frequency converter (I/F converter) 430.

The microcontroller 410 includes software program code or programmable logic which, when executed, causes the microcontroller 410 to transmit a signal to the DAC 420, where the signal is representative of a voltage level or value that is to be applied to the sensor 355. The DAC 420 receives the signal and generates the voltage value at the level instructed by the microcontroller 410. In aspects of the disclosure, the microcontroller 410 may change the representation of the voltage level in the signal frequently or infrequently. Illustratively, the signal from the microcontroller 410 may instruct the DAC 420 to apply a first voltage value for one second and a second voltage value for two seconds.

The sensor 355 may receive the voltage level or value. In an aspect of the disclosure, the counter electrode 365 may receive the output of an operational amplifier which has as inputs the reference voltage and the voltage value from the DAC 420. The application of the voltage level causes the sensor 355 to create a sensor signal indicative of a concentration of a physiological characteristic being measured. In an aspect of the disclosure, the microcontroller 410 may measure the sensor signal (e.g., a current value) from the working electrode. Illustratively, a sensor signal measurement circuit 431 may measure the sensor signal. In an aspect of the disclosure, the sensor signal measurement circuit 431 may include a resistor and the current may be passed through the resistor to measure the value of the sensor signal. In an aspect of the disclosure, the sensor signal may be a current level signal and the sensor signal measurement circuit 431 may be a current-to-frequency (I/F) converter 430. The I/F converter 430 may measure the sensor signal in terms of a current reading, convert it to a frequency-based sensor signal or EIS signal, and transmit the frequency-based sensor signal or EIS signal to the microcontroller 410. In aspects of the disclosure, the microcontroller 410 may be able to receive frequency-based sensor signals easier than non-frequency-based sensor signals. The microcontroller 410 receives the sensor signal, whether frequency-based or non-frequency-based, and determines a value for the physiological characteristic of a subject, such as a blood glucose level. The microcontroller 410 may include program code, which when executed or run, is able to receive the sensor signal and convert the sensor signal to a physiological characteristic value.

In one aspect of the disclosure, the microcontroller 410 may convert the sensor signal to a blood glucose level. While converting the sensor signal to a blood glucose value, the microcontroller 410 may use one or more models, which are specific ways to use the sensor signal to calculate the blood glucose value. In some aspects, the microcontroller 410 may utilize measurements (e.g., sensor signals and electrochemical impedance spectroscopy (EIS) signals from the sensor 355) stored within an internal memory in order to determine the blood glucose level of the subject. In some aspects, the microcontroller 410 may utilize measurements stored within a memory external to the microcontroller 410 to assist in determining the blood glucose level of the subject.

After the physiological characteristic value is determined by the microcontroller 410, the microcontroller 410 may store measurements of the physiological characteristic values for a number of time periods. For example, a blood glucose value (BG) may be sent to the microcontroller 410 from the sensor every second or five seconds, and the microcontroller may save sensor measurements for five minutes or ten minutes of BG readings. The microcontroller 410 may transfer the measurements of the physiological characteristic values to a display on the sensor electronics device 360. For example, the sensor electronics device 360 may be a monitor which includes a display that provides a blood glucose reading for a subject. In one aspect of the disclosure, the microcontroller 410 may transfer the measurements of the physiological characteristic values to an output interface of the microcontroller 410. The output interface of the microcontroller 410 may transfer the measurements of the physiological characteristic values, e.g., blood glucose values, to an external device, e.g., an infusion pump, a combined infusion pump/glucose meter, a computer, a personal digital assistant, a pager, a network appliance, a server, a cellular phone, or any computing device.

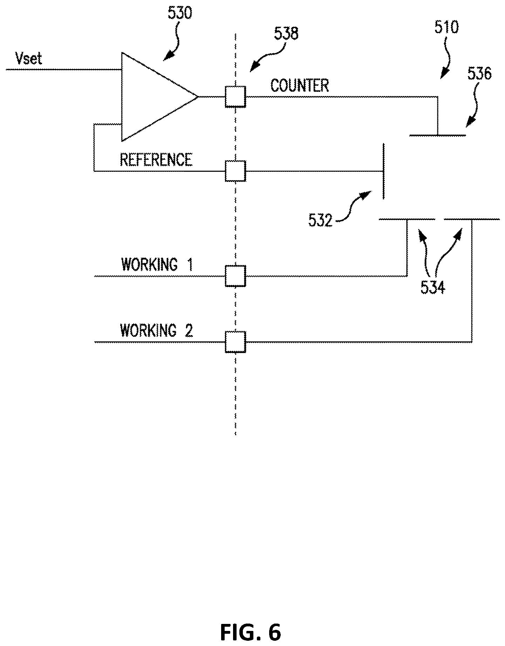

FIG. 6 illustrates an electronic block diagram of the sensor electrodes and a voltage being applied to the sensor electrodes according to one aspect of the present disclosure. In the aspect illustrated in FIG. 6, an op amp 530 or other servo controlled device may connect to sensor electrodes 510 through a circuit/electrode interface 538. The op amp 530, utilizing feedback through the sensor electrodes, attempts to maintain a prescribed voltage (what the DAC may desire the applied voltage to be) between a reference electrode 532 and a working electrode 534 by adjusting the voltage at a counter electrode 536. Current may then flow from a counter electrode 536 to a working electrode 534. Such current may be measured to ascertain the electrochemical reaction between the sensor electrodes 510 and the biomolecule of a sensor that has been placed in the vicinity of the sensor electrodes 510 and used as a catalyzing agent. The circuitry disclosed in FIG. 6 may be utilized in a long-term or implantable sensor or may be utilized in a short-term or subcutaneous sensor.

In a long-term sensor aspect, where a glucose oxidase (GOx) enzyme is used as a catalytic agent in a sensor, current may flow from the counter electrode 536 to a working electrode 534 only if there is oxygen in the vicinity of the enzyme and the sensor electrodes 510. Illustratively, if the voltage set at the reference electrode 532 is maintained at about 0.5 volts, the amount of current flowing from the counter electrode 536 to a working electrode 534 has a fairly linear relationship with unity slope to the amount of oxygen present in the area surrounding the enzyme and the electrodes. Thus, increased accuracy in determining an amount of oxygen in the blood may be achieved by maintaining the reference electrode 532 at about 0.5 volts and utilizing this region of the current-voltage curve for varying levels of blood oxygen. Different aspects of the present disclosure may utilize different sensors having biomolecules other than a glucose oxidase enzyme and may, therefore, have voltages other than 0.5 volts set at the reference electrode. A stabilization period is needed for many sensors in order for the sensor 510 to provide accurate readings of the physiological parameter of the subject. During the stabilization period, the sensor 510 does not provide accurate blood glucose measurements. Users and manufacturers of the sensors may desire to improve the stabilization timeframe for the sensor so that the sensors can be utilized quickly after insertion into the subject's body or a subcutaneous layer of the subject.

Even after stabilization timeframe or during initial implantation or insertion of the sensor 510, the sensor 510 may provide inaccurate sensor signals due to signal noises in the sensor, electrochemical byproducts caused by oxidation and reduction, or a foreign body response that prevents oxygen from the interstitial tissue from reaching the chemistry layers on top of the working electrode, specifically the glucose oxidase layer. When the inaccuracy of the sensor signal becomes higher than a threshold, the working electrode 534 should be replaced and the user of the sensor needs to be notified. To lengthen the lifetime of the working electrode 534, an agent is coated over the working electrode 534. In an aspect, the agent is coated on a secondary polyimide flex that is placed adjacent to the primary flex which has the working electrode. The purpose of the agent is to inhibit the foreign body response so that sufficient oxygen from the interstitial tissue reaches the chemistry layers deposited above the working electrode. Such agent may be dexamethasone, dexamethasone phosphate, dexamethasone acetate, corticosteroids, NSAIDs, antifibrotic agents, and/or siRNA. With the presence of this coating of the agent, the lifetime of working electrode 534 can be lengthened from about a week to about 16 days or more.

In an alternative aspect, the agent may be coated on the primary flex, which has the working electrode. The agent may be coated in the top portion of the primary flex and the working electrode may be on the bottom portion of the primary flex. In an aspect, the agent may be coated in the bottom portion of the primary flex and the working electrode may be on the top portion. In another aspect, the agent and the working electrode may be positioned at different places from each other on the primary flex. Regardless of positions of the agent and the working electrode, when the glucose sensor is mounted on the user, the primary flex enters into the body of the user so that the agent and the working electrode also enter into the body.

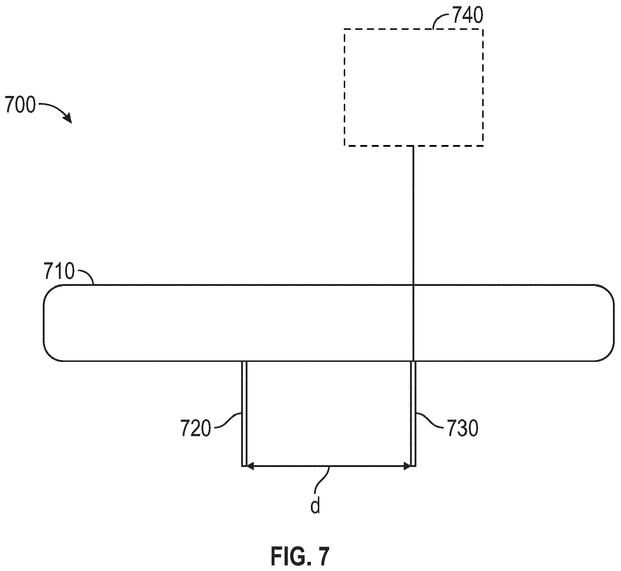

FIG. 7 illustrates a block diagram of an all-in-one glucose sensor system 700 according to aspects of the present disclosure. The glucose sensor system 700 may include a housing 710, a glucose sensor 720, which is affixed on a substrate, and an optional insulin delivery cannula 730. Electronic circuits for a glucose sensor may be fabricated within the housing 710, and one or more electrodes, which are subcutaneously into a body of the user, may be placed on the glucose sensor 720. The optional insulin delivery cannula 730 of the glucose sensor system 700 is also subcutaneously inserted into the body of the user, and insulin may be injected therethrough. In this regard, an insulin pump 740 may be optionally incorporated into the glucose sensor system 700. The insulin pump 740 may be connected to an insulin storage storing insulin and may pump insulin out to the user via the optional insulin delivery cannula 730.

The distance "d" between the glucose sensor 720 and the optional insulin delivery cannula 730 may affect how excipients, which are part of the injected insulin to enhance stability of insulin, affect the glucose sensor's readings. The closer the distance "d" is, the faster and greater the effects of the excipients to the glucose sensor's readings are. In a case where the optional insulin delivery cannula 730 is integrated within the glucose sensor system 700, the distance "d" may be short, thereby the glucose sensor system 700 can be made small enough to be portably carried. In an aspect, the distance "d" may be less than or equal to 25 mm.

The excipients may be a combination of peroxide (H₂O₂), phenol (C₆H₆O), M-cresol (CH₃C₆H₄(OH)), glycerol (C₃H₈O₃), zinc (Zn), zinc oxide (ZnO), disodium phosphate (Na₂HPO₄), sodium chloride (NaCl), sodium hydroxide (NaOH), hydrogen chloride (HCl), niacinamide (C₆H₆N₂O), and/or arginine hydrochloride (C₆H₁₃ClN₄O₂), among others.

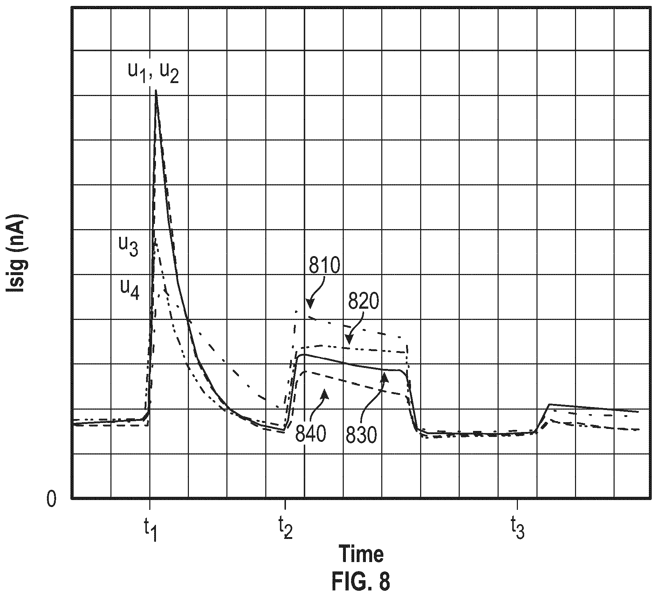

The effects of excipients of insulin on the current (Isig), which is indicative of concentration of glucose, at the working electrode of the glucose sensor are illustrated as the curves 810-840 of FIG. 8 in various scenarios. The vertical axis represents amplitudes of Isig in unit of nano amps (nA) and the horizontal axis represents time in unit of minutes. During the example scenarios, in the presence of 100 mg/dL concentration of glucose, an insulin placebo, which includes not insulin but excipients of insulin, is injected at t₁, and 3 ppm peroxide is introduced at t₂. At t₃, a new solution is prepared with the presence of 100 mg/dL concentration of glucose, and another insulin placebo is introduced during the scenarios.

The curves 810-840 are obtained at concentrations of insulin of 2.5%, 2.0%, 1.0%, and 0.5%, respectively. At the introduction of the first placebo at t₁, all curves 810-840 show a sudden increase at t₁. Such increase is a false increase in glucose readings, because there is no change in the glucose level. The sudden increase may be detected by comparing an amount of increase during a predetermined period. For example, when the increase in Isig is greater than or equal to a predetermined threshold (e.g., 10 nA) during the predetermined period (e.g., 5 minutes), it is determined that there is a sudden increase or change.

The apexes of the curves 810-840 are about u₁, u₂, u₃, and u₄ nA, respectively, where u₁ >= u₂ > u₃ > u₄. Thus, the apex of Isig may be dependent upon a concentration of insulin or, in other words, a concentration of excipients of insulin.

During the period from t₁ to t₂ when 3 ppm peroxide is introduced, the effect of the excipients subdues the Isig. However, after introduction of 3 ppm peroxide, which is one of excipients of insulin and is one of main excipients which causes the false increase in Isig values, the curves 810-840 also show another sudden increase during the predetermined period, even though the increase at this time is smaller than the increase at the injection of the first placebo. As shown in these example scenarios, the effects of an insulin injection in Isig may decrease along consecutive introductions of excipients of insulin.

The apexes of the curves 810-840 are different from each other. Further, the apex of the curve 810 is the highest as the concentration of insulin for the curve 810 is the highest, while the apex of the curve 840 is the lowest as the concentration of insulin for the curve 840 is the lowest. Thus, the apex of Isig may be dependent upon a concentration of insulin.

Starting anew at t₂ with the presence of 100 mg/dL concentration of glucose, the curves 810-840 shows substantially flat lines, which is indicative of the 100 mg/dL concentration of glucose. At about t₃, another placebo, which is different from the first placebo, is introduced, thereby causing another false increase in Isig.

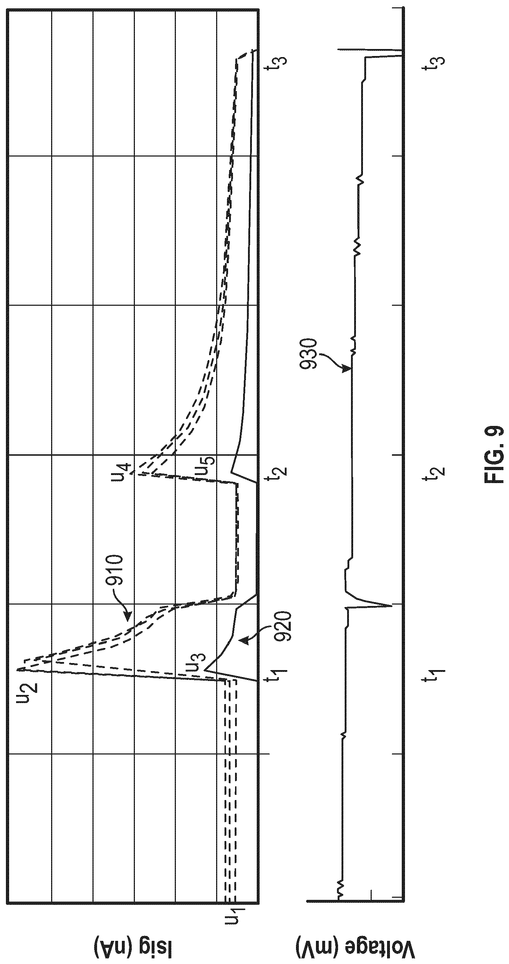

In accordance with aspects of the present disclosure, to compensate for the false increase in Isig at the working electrode based on introduction or injection of insulin, presence of excipients, which are preservatives of insulin, are detected. FIGS. 9-18 illustrate various relationships between signals (e.g., Isig at a working electrode and Isig at a background electrode) of the glucose sensor. As used herein, the term "background electrode" refers to an electrode of the glucose sensor which may be used to detect presence of excipients. Referring again to FIG. 7, both the working electrode and the background electrode (not shown) may be located in the glucose sensor 720.

Provided by FIG. 9 are graphical illustrations of scenarios of insulin injections at t₁ and at t₂ from the start of the scenarios according to aspects of the present disclosure. The top graph shows an Isig curve 910 measured at the working electrode of the glucose sensor and the Isig curve 920 measured at the background working electrode of the glucose sensor. The vertical axis represents amplitudes of Isig in the unit of nA and the horizontal axis represents a time of one day.

During the full one day, 40 mg/dL concentration of glucose is present in the scenarios. Before the first injection of insulin at t₁, the Isig at the working electrode stays at u₁ nA, which is indicative of 40 mg/dL concentration of glucose, while Isig at the background electrode stays zero, meaning that excipients of insulin are not detected. At t₁, 2.5 mL of insulin, of which concentration is 0.5%, is introduced or injected for a first injection period (e.g., 30 minutes). Due to addition of the excipients in the insulin, Isig at the working electrode increases to u₂ nA and Isig at the background electrode also increases to u₃ nA. Such increases are considered as sudden increases because the increases are greater than the predetermined threshold during the predetermined period, and are false because the level of glucose stays the same. From t₁ to t₂, both Isigs become stabilized to u₁ because the excipients are dispersed and the effect thereof becomes correspondingly lowered.

Now at t₂, another injection of 2.5 mL insulin, of which concentration is 0.5%, is made for a second injection period, which is longer than the first injection period. Due to the slower injection of the same concentration of insulin, the apex of Isig at the working electrode hits u₄ nA, which is lower than the apex u₂ during the first injection, and the apex of Isig at the background electrode is u₅ nA, which is lower than the apex u₃ during the first injection. Further, due to the slower injection during the second injection period, Isig at the working electrode and Isig at the background electrode take a long period to stabilize than that during the first injection. In an aspect, the relationship between Isig at the working electrode and Isig at the background electrode may be linear during the first and second injections of insulin. The linear relationship may be expressed by a ratio. In another aspect, the linear ratio between Isig at the working electrode and Isig at the background electrode during the first injection may be substantially the same during the period of the first injection, and likewise, the linear ratio between Isig at the working electrode and Isig at the background electrode during the second injection may be substantially the same during the period of the second injection. However, the ratio during the first injection may be higher than the ratio during the second injection.

In aspects, the linear ratio may depend on the number of injections. In other words, the linear ratio may linearly or exponentially decrease along the number of insulin injections. Thus, a ratio profile may be stored in the glucose sensor and provide a change of the linear ratio along the number of injections of insulin so as to properly compensate for the false increase in the Isig at the working electrode during injections of insulin.

For example, compensation may be implemented by the following equation (1): ${Isig}_{COMP} = {Isig}_{WE 1} - α * {Isig}_{WE 2}$ where Isig_{COMP} is the compensated Isig_{WE1}, Isig_{WE1} is the Isig at the working electrode, Isig_{WE2} is the Isig at the background electrode, and α is the linear ratio between Isig_{WE1} and Isig_{WE2}.

In a case when a cannula, via which insulin is injected, is included in the glucose sensor, the linear ratio may also depend on a distance (e.g., "d" in FIG. 7) between the background electrode and the cannula.

The bottom graph of FIG. 9 shows a voltage curve 930 across the background electrode, and the vertical axis is a voltage in unit of millivolts (mV). As shown in the voltage curve 930, the voltage does not increase or decrease at the start of or during the first and second insulin injections and thus, may not be used as an indicator for detection of the insulin injections.

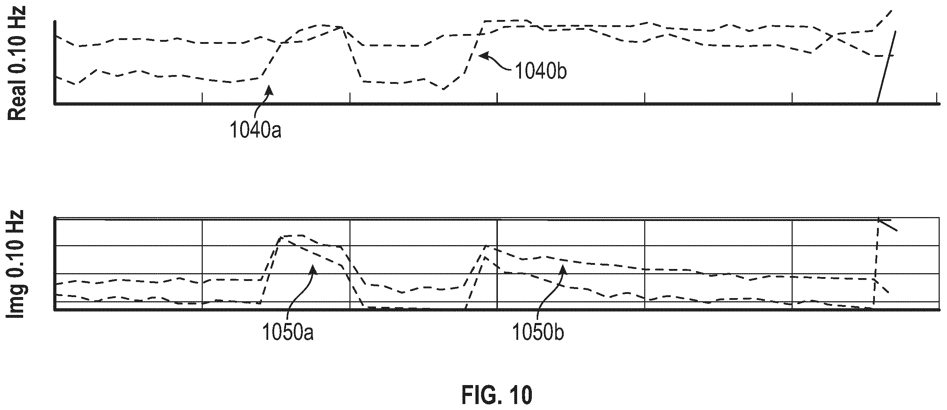

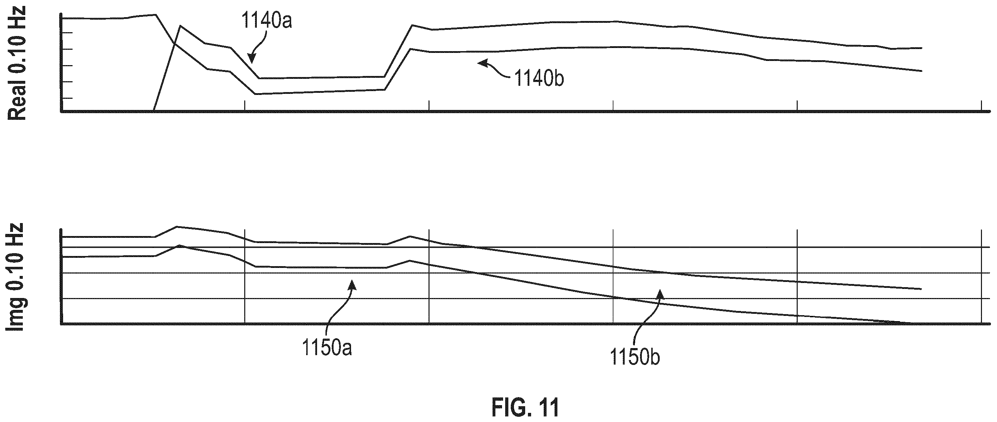

FIGS. 10 and 11 show real and imaginary impedance at the working and background electrodes during the scenarios of FIG. 9 according to aspects of the present disclosure. The top graph of FIG. 10 shows two curves 1040a and 1040b of electrochemical impedance spectroscopy (EIS) signals, which are measured at the working electrode during the two scenarios. In particular, the curves 1040a and 1040b are real impedance at 0.1 Hz in unit of 10⁵ Ω. The curve 1040a shows an increase in the real impedance of the EIS signal during the first insulin injection, and the curve 1040b shows an increase in the real impedance of the EIS signal during the second insulin injection. However, the curve 1040a does not show an apparent increase in the real impedance during the second insulin injection and the curve 1040b does not show an apparent increase in the real impedance during the first insulin injection. Thus, the real impedance at 0.1 Hz at the working electrode may not be mainly or solely used to detect presence of excipients of insulin but may be used to confirm detection of presence of excipients.

The bottom graph of FIG. 10 shows two curves 1050a and 1050b of EIS signals, which are measured at the working electrode during the two scenarios. In particular, the curves 1050a and 1050b are imaginary impedance at 0.1 Hz in unit of 10⁵ Ω. The curves 1050a and 1050b show increases in the imaginary impedance of EIS signal during the first and second insulin injections during the predetermined period. The imaginary impedance of the EIS signal at 0.1 Hz from the working electrode may be used to detect the injection of insulin or presence of excipients of insulin. When the increase in the imaginary impedance at 0.1 Hz is greater than a threshold impedance (e.g., 5*10⁶ Ω) during the predetermined period (e.g., 5 minutes), the presence of excipients of insulin may be determined. In this way, the imaginary impedance of the EIS signal at 0.1 Hz from the working electrode may be used to confirm the injection of insulin or presence of excipients of insulin.

The top graph of FIG. 11 shows two curves 1140a and 1140b of EIS signals, which are measured at the background electrode during the two scenarios. In particular, the curves 1140a and 1140b are real impedance at 0.1 Hz in unit of 10⁵ Ω. The curve 1140a shows an increase in the real impedance of the EIS signal during the first insulin injection. Even though the curve 1140b does not show an apparent increase in the real impedance of the EIS signal during the first insulin injection, the increase in impedance may be greater than the threshold impedance because the unit is 10⁵ Ω. The threshold impedance may be between 0.5*10⁶ and 5*10⁶ Ω. The curve 1140a also shows an increase in the real impedance during the second insulin injection and the curve 1140b also shows an increase in the real impedance during the second insulin injection. Thus, the real impedance at 0.1 Hz at the background electrode may be used to detect presence of excipients of insulin and to confirm detection of presence of the excipients.

The bottom graph of FIG. 11 shows two curves 1150a and 1150b of EIS signals, which are measured at the background electrode during the two scenarios. In particular, the curves 1150a and 1150b are imaginary impedance at 0.1 Hz in unit of 10⁵ Ω. The curves 1150a and 1150b show minor increases, which may be greater than the threshold impedance, in the imaginary impedance of EIS signal during the first and second insulin injections, while the curves 1150a and 1150b show generally decreases in imaginary impedance. Even the increase appears minor, the increase may be substantial because of the unit of magnitude, 10⁵ Ω. Thus, the imaginary impedance of the EIS signal at 0.1 Hz from the background electrode may be used to detect the injection of insulin or presence of excipients of insulin. Further, the imaginary impedance of the EIS signal at 0.1 Hz from the background electrode may be used to confirm the injection of insulin or presence of excipients of insulin.

In summary for FIGS. 9-11, the presence of excipients of insulin may be detected by Isig from the background electrode and may be confirmed by a change in real or imaginary impedance at 0.1 Hz of the EIS signal from one of the working and background electrodes during the predetermined period.

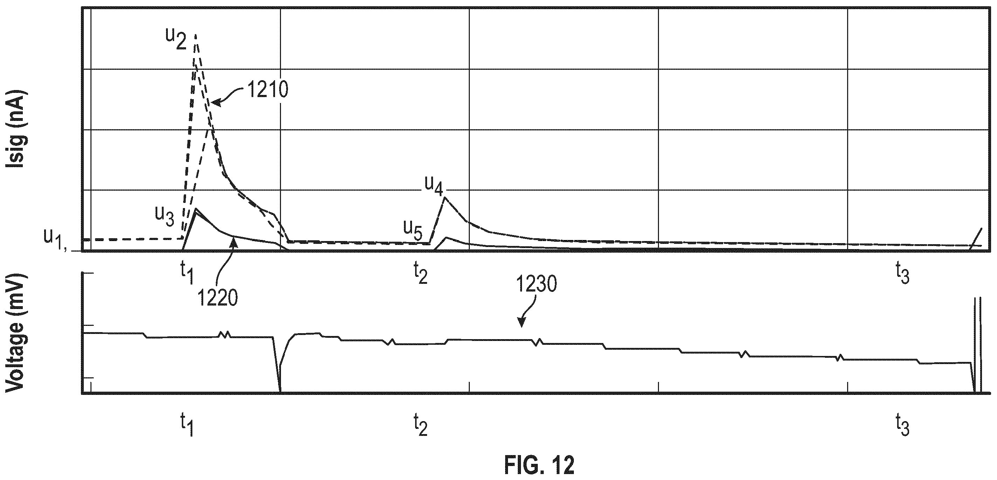

Provided by FIG. 12 are graphical illustrations showing scenarios of insulin injections at t₁ and at t₂ from the start of the scenarios according to aspects of the present disclosure. The top graph shows an Isig curve 1210 measured at the working electrode of the glucose sensor and the Isig curve 1220 measured at the background working electrode of the glucose sensor. The vertical axis represents amplitudes of Isig in the unit of nA and the horizontal axis represents time.

During the scenarios, 40 mg/dL concentration of glucose is present. Thus, Isig at the working electrode stays at a constant level u₁, which is indicative of 40 mg/dL concentration of glucose, while Isig at the background electrode stays zero, meaning that no excipients of insulin are detected. At t₁, 2.5 mL of insulin, of which concentration is 2.5%, is introduced or injected for a first injection period (e.g., 30 minutes). Due to the presence of excipients in the insulin, Isig at the working electrode increases to u₂ nA and Isig at the background electrode also increases to u₃ nA during the predetermined period. Such increases are false because the level of glucose stays the same. For the next 1 hour or until t₂, both Isigs become stabilized to the original value u₁ because the excipients are dispersed and the effect thereof becomes diminished. The apex u₂ of the Isig curve 1210 is about 2 or 3 times the apex u₂ of the Isig curve 910 of FIG. 9, and the apex u₃ of the Isig curve 1220 is about 2 or 3 times the apex u₃ of the Isig curve 920 of FIG. 9. Even though the concentration of insulin from the scenarios of FIG. 9 to the scenarios of FIG. 12 is increased 5 times, the ratio of apexes between the scenarios of FIG. 9 and the scenarios of FIG. 12 is not linearly increased. The relationship between the concentration of insulin and the apex of Isig during the first injection may be stored as a ratio profile in a memory of the glucose sensor, and based on the relationship, the apex of Isig at the background electrode may be used as an indication of the concentration of insulin. Further, the relationship between the apex of Isig at the working electrode and the apex of Isig at the background electrode at different concentration of insulin may be incorporated into the ratio profile.

Now at t₂, another injection of 2.5 mL insulin, of which concentration is 2.5%, is made over a second injection period, which is longer than the first injection period. Due to the slower injection of the same concentration of insulin than the first injection, the apex of Isig at the working electrode is u₄ nA, while the apex of Isig at the background electrode is u₅ nA. Further, due to the slower injection during the second injection, Isig at the working electrode and Isig at the background electrode take longer hours to be stabilized than that of the first injection. In an aspect, the relationship between Isig at the working electrode and Isig at the background electrode may be linear during the first and second injections of insulin. The linear relationship may be expressed by a ratio. In another aspect, the linear ratio between Isig at the working electrode and Isig at the background electrode during the first injection may be substantially the same during the period of the first injection, and likewise, the linear ratio between Isig at the working electrode and Isig at the background electrode during the second injection may be substantially the same during the period of the second injection. However, the ratio during the first injection may be higher than the ration during the second injection.

In aspects, the linear ratio may depend on the number of injections. In other words, the linear ratio may linearly or exponentially decrease along the number of insulin injections.

In a case when a cannula, via which insulin is injected, is integrated in the glucose sensor, the linear ratio may also depend on a distance (e.g., "d" in FIG. 7) between the flex, where the background electrode is fixed or imprinted, and the cannula.

The bottom graph shows a voltage curve 1230 of a voltage across the background electrode, and the vertical axis is a voltage in unit of millivolts (mV). As shown in the voltage curve 1230, the voltage does not increase or decrease at the start of or during the first and second insulin injections and thus, may not be used as an indicator for detection of the insulin injections.

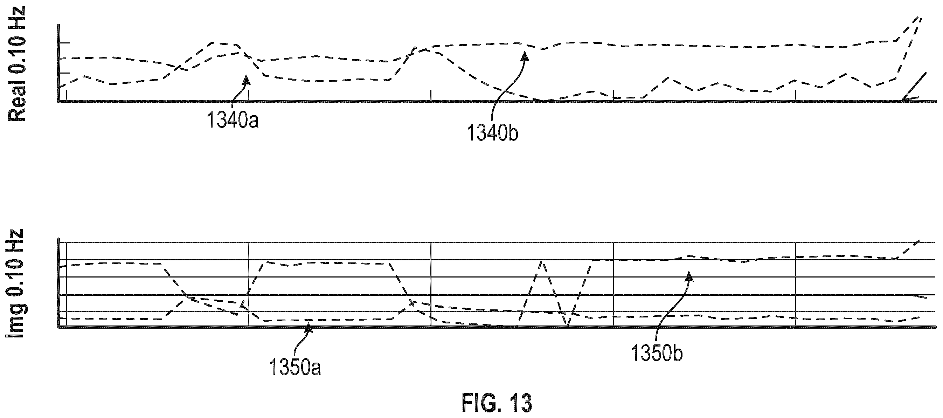

The top graph of FIG. 13 shows two curves 1340a and 1340b of EIS signals, which are measured at the working electrode during two scenarios. In particular, the curves 1340a and 1340b are real impedance at 0.1 Hz in unit of 10⁵ Ω. The curves 1340a and 1340b show increases in real impedance of the EIS signal during the first insulin injection. The curve 1340b shows an increase in the real impedance during the second injection, while the curve 1340a does show a minor increase, which is greater than the threshold impedance during the predetermined period, in the real impedance during the second insulin injection. Thus, the real impedance at 0.1 Hz at the working electrode may be used to detect presence of excipients of insulin. Further, based on the two scenarios, the real impedance of the EIS signal at 0.1 Hz from the working electrode may be used to confirm the injection of insulin or presence of excipients of insulin after detection of such.

The bottom graph of FIG. 13 shows two curves 1350a and 1350b of EIS signals, which are measured at the working electrode during the two scenarios. In particular, the curves 1350a and 1350b are imaginary impedance at 0.1 Hz in unit of 10⁵ Ω. The curve 1350a shows increases in the imaginary impedance of EIS signal during the first and second insulin injections, while the curve 1350b shows decreases during the first and second insulin injections. Thus, the imaginary impedance of the EIS signal at 0.1 Hz from the working electrode may not be mainly or solely used to detect the presence of excipients of insulin but may be used to confirm the injection of insulin or presence of excipients of insulin.

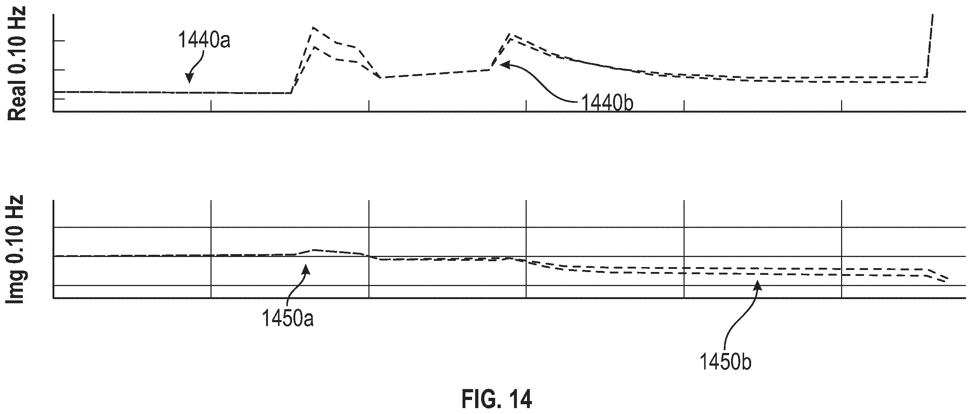

The top graph of FIG. 14 shows two curves 1440a and 1440b of EIS signals, which are measured at the background electrode during the two scenarios. In particular, the curves 1440a and 1440b are real impedance at 0.1 Hz in unit of 10⁵ Ω. Both curves 1440a and 1440b show increases, which are greater than the threshold impedance during the predetermined period, in the real impedance of the EIS signal during the first and second insulin injections. Thus, the real impedance at 0.1 Hz at the background electrode may be used to detect presence of excipients of insulin.

The bottom graph of FIG. 14 shows two curves 1450a and 1450b of EIS signals, which are measured at the background electrode during the two scenarios. In particular, the curves 1450a and 1450b are imaginary impedance at 0.1 Hz in unit of 10⁵ Ω. The curves 1450a and 1450b show minor bumps in the imaginary impedance of EIS signal during the first and second insulin injections, while the curves 1450a and 1450b show general decreases in imaginary impedance. Even though the increase appears minor, the increase may be substantial because of the unit of magnitude, 10⁵. Thus, the increase during the predetermined period in the imaginary impedance of the EIS signal at 0.1 Hz from the background electrode may be used to detect the injection of insulin or presence of excipients of insulin. Further, the increase during the predetermined period in the imaginary impedance of the EIS signal at 0.1 Hz from the background electrode may be used to confirm the injection of insulin or presence of excipients of insulin.

In summary for FIGS. 12-14, the presence of excipients of insulin may be detected by Isig at the background electrode and may be confirmed by the real or imaginary impedance at 0.1 Hz of the EIS signal from the working and background electrodes.

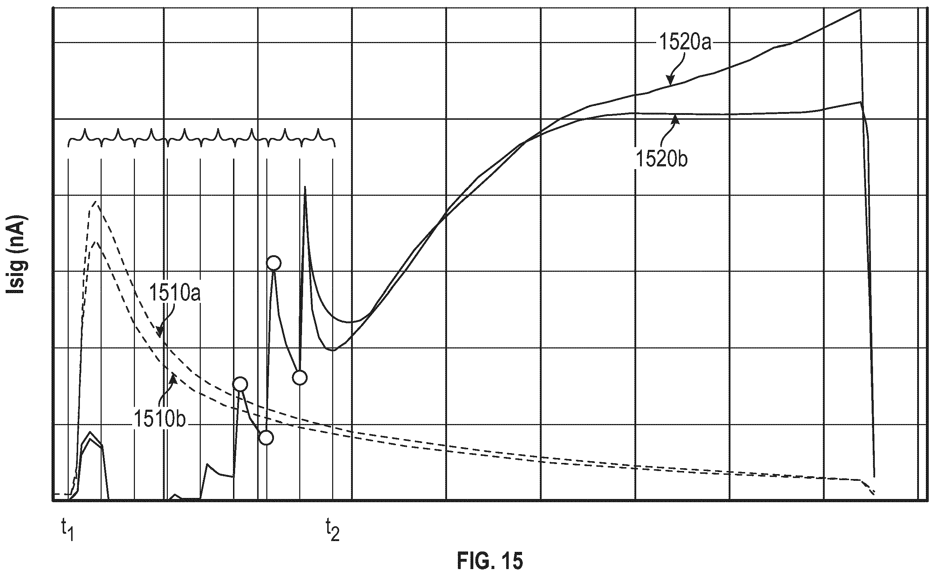

Provided by FIG. 15 are graphical illustrations showing scenarios on changes to voltage across the background electrode according to aspects of the present disclosure. Curves 1510a and 1510b are Isig curves measured at the working electrode and curves 1520a and 1520b are Isig curves measured at the background electrode. No glucose is present during the scenarios, while 0.5 % insulin is introduced or injected at t₁. As such, all the curves 1510a, 1510b, 1520a, and 1520b are substantially zero until t₁. The injection is made for from t₁.

After the introduction of insulin at t₁, the Isig curves 1510a and 1510b are abruptly increased and the increases are false because there is no glucose present. Similarly, the Isig curves 1520a and 1520b are abruptly increased at the background electrode. Right after the injection of insulin, 535 mV of voltage is applied to the background electrode for every unit period (e.g., 5 minutes or 10 minutes). During the application of 535 mV, the Isig curves 1520a and 1520b show proportionally similar increases in the Isig curves 1510a and 1510b, meaning that Isig at the working electrode and Isig at the background electrode have a linear relationship therebetween in the presence of insulin excipients.

Thereafter, 200 mV, 300 mV, 400 mV, 500 mV, 600 mV, 700 mV, and 800 mV are applied to the background electrode for every unit period in order. During application of 200, 300, and 400 mV to the background electrode, the Isig curves 1520a and 1520b are substantially zero. However, during application of 500 mV, the Isig curves 1520a and 1520b show an increase and a following decrease which appears to be proportionally similar to the decrease shown in the Isig curves 1510a and 1510b of the working electrode. On the other hand, during the application of 600-800 mV, both increase and decrease of the Isig curves 1520a and 1520b are abrupt. Thus, the range of voltage from 500 mV to 600 mV to the background electrode may be used for detecting the presence of excipient of insulin. In an aspect, 535 mV may be applied to the background electrode to detect presence of excipient of insulin.

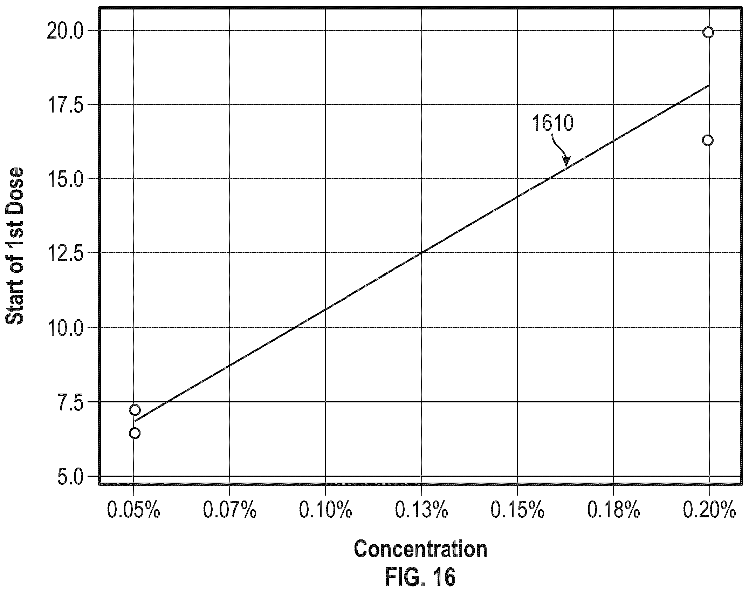

Provided by FIG. 16 is an example of a linear relationship 1610 between Isig signal at the background electrode and insulin concentration at the start of the first injection of insulin according to aspects of the present disclosure. The vertical axis represents peak amplitudes of Isig in unit of nA, and the horizontal axis represents insulin concentration in unit of percentage. The linear relationship may be expressed as a linear function (1), for example: $Peak of Isig = {α}_{1} + {s}_{1} * Concentration$

For example, when 0.05% concentration of insulin is firstly injected, the peak value of Isig at the background electrode may be calculated to be, based on the linear relationship (1) with α₁ and s₁, 6.8 nA, and when 0.2% concentration of insulin is injected, the peak value of Isig at the background electrode may be calculated to be 18.14 nA. The values of α₁ and s₁ at the start of the first injection may be predetermined depending on the characteristics of the glucose sensor.

In aspects, when the unknown concentration of insulin is firstly injected, the concentration of insulin may be calculated based on the linear relationship between the apex or peak value of Isig signal at the background electrode and insulin concentration at the start of the injection of insulin. The linear relationship may be a function of the number of injections.

For example, in a case when Isig at the background electrode is measured at the start of the first injection, the concentration of insulin may be calculated by the following equation (2): $Concentration = \frac{Peak of Isig - {α}_{1}}{{s}_{1}}$

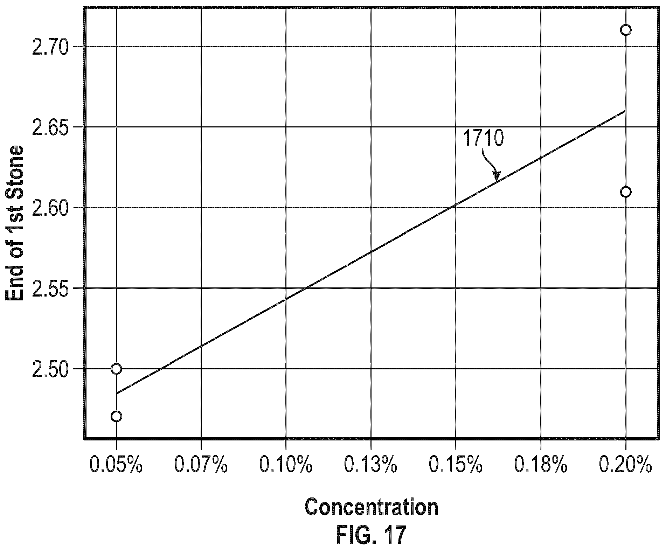

Provided by FIG. 17 is a linear relationship 1710 between Isig signal at the background electrode and insulin concentration at the end of the first injection of insulin according to aspects of the present disclosure. The vertical axis represents amplitudes of Isig in unit of nA, and the horizontal axis represents insulin concentration in unit of percentage. The linear relationship may be expressed as a linear function (3), for example: $Isig = {α}_{2} + {s}_{2} * Concentration$

For example, after 0.05% concentration of insulin is completely injected, the value of Isig at the background electrode may be calculated, based on the relationship (2), to be about 2.49 nA, and when 0.2% concentration of insulin completely injected, the value of Isig at the background electrode may be calculated, based on the relationship (2), to be about 2.66 nA. The values of α₂ and s₂ may be predetermined depending on the characteristics of the glucose sensor.

In aspects, when the unknow concentration of insulin is firstly injected, the concentration of insulin may be calculated based on the linear relationship (3) between Isig signal at the background electrode and insulin concentration at the end of the injection of insulin. The linear relationship may be a function of the number of injections.

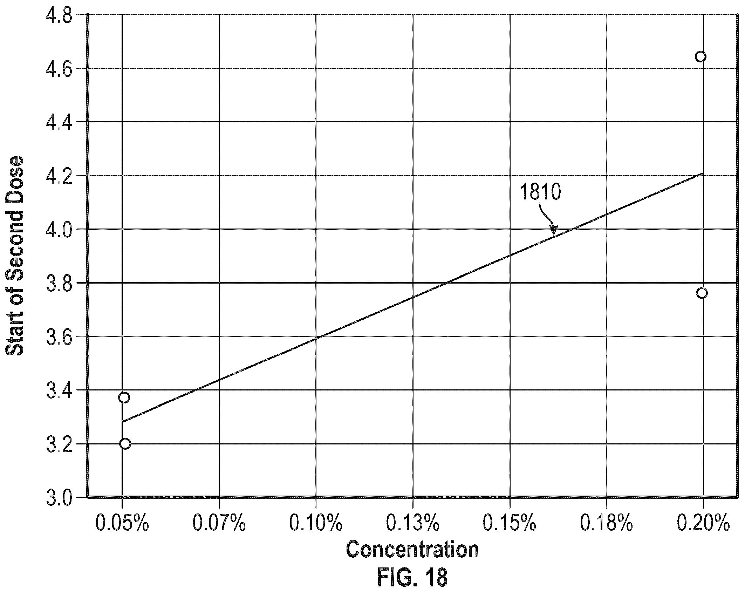

Provided by FIG. 18 is a linear relationship 1810 between Isig signal at the background electrode and insulin concentration at the start of the second injection of insulin according to aspects of the present disclosure. The vertical axis represents peak amplitudes of Isig in unit of nA, and the horizontal axis represents insulin concentration in unit of percentage. The linear relationship may be expressed as a linear function (4), for example: $Peak of Isig = {α}_{3} + {s}_{3} * Concentration$

Specifically, when 0.05% concentration of insulin is secondly injected, the peak value of Isig at the background electrode may be calculated based on the linear relationship (4) to be about 3.26 nA, and when 0.2% concentration of insulin is secondly injected, the peak value of Isig at the background electrode may be calculated based on the linear relationship (4) to be about 4.20 nA. The values of α₃ and s₃ may be predetermined depending on the characteristics of the glucose sensor.

Based on these linear relationships, when an unknown concentration of insulin is injected at the first, second, third or any number of times, the concentration of the insulin may be calculated based on the peak value of the Isig from the background electrode. In aspects, the glucose sensor may include a storage storing these relationships between the peak values and the number of insulin injections, and may be able to estimate the concentration of insulin. For example, when the peak value of Isig at the background electrode is measured to be 4.20 nA after the start of the second injection of insulin, the concentration of insulin may be estimated as 0.2% based on the following equation (5): $Concentration = \frac{Peak of Isig - {α}_{3}}{{s}_{3}}$

As shown in FIGS. 16 and 18, the peak value of Isig at the background electrode at the start of the second injection is smaller than the peak value at the start of the first injection. Thus, the peak values of Isig decreases along the number of injections of insulin. The decreasing pattern of peak values in the Isig at the start of each injection may be stored in the ratio profile. Likewise, the decreasing pattern of peak values in the Isig at the end of each injection may be stored in the ration profile.

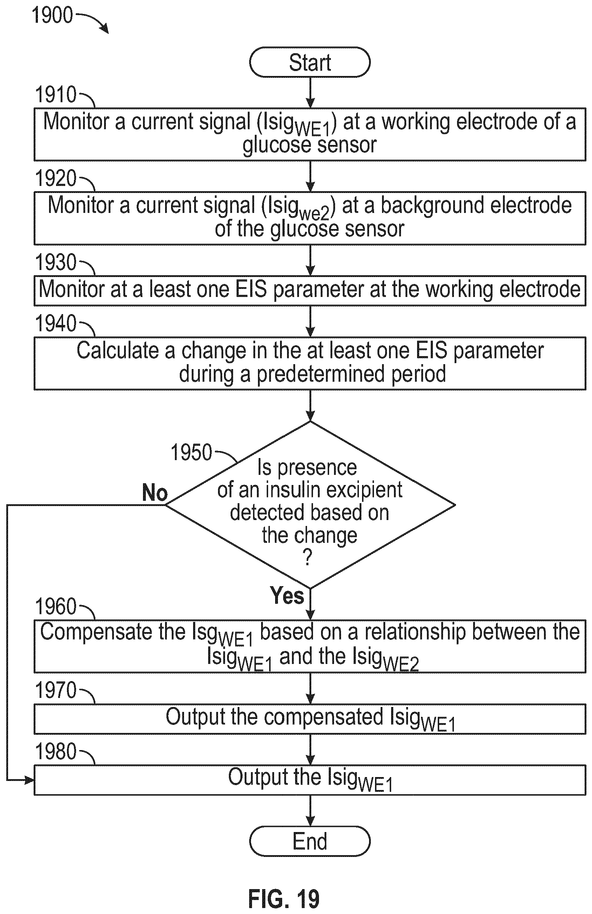

FIG. 19 shows a flowchart of a method 1900 for detecting presence of an excipient in insulin by a glucose sensor in accordance with aspects of the present disclosure. The glucose sensor may include a working electrode, a background electrode, and a processor. The method 1900 starts by monitoring a current signal (Isig_{WE1}) at the working electrode by the controller of the glucose sensor in step 1910 and monitoring a current signal (Isig_{WE2}) at the background electrode by the controller in step 1920.

The processor further monitors an electrochemical impedance spectroscopy (EIS) signal at the working electrode in step 1930. A change in the EIS signal is of at least one parameter, which is a real or imaginary impedance at 0.1 Hz. A change is the EIS signal is calculated by the controller in step 1940. In an aspect, the EIS signal may be measured at the background electrode in step 1930.

In step 1950, in a case where there is no change in the EIS signal during a predetermined period, it is determined that there is no presence of excipients of insulin, and in a case where there is a change, which is greater than a predetermined threshold, in the EIS signal, it is determined that there are excipients of insulin.

In a case when it is determined that there is no presence of excipients of insulin, Isig_{WE1}, which is indicative of a level of insulin, is output in step 1980.

In a case when it is determined that there are excipients of insulin, Isig_{WE1} is compensated based on a linear relationship between the Isig_{WE1} and Isig_{WE2} in step 1980. As described above with respect to FIG. 9, the compensated Isig_{WE1} (Isig_{COMP}) is equal to: ${Isig}_{COMP} = {Isig}_{WE 1} - α * {Isig}_{WE 2} .$ The Isig_{COMP} is then output in step 1970.

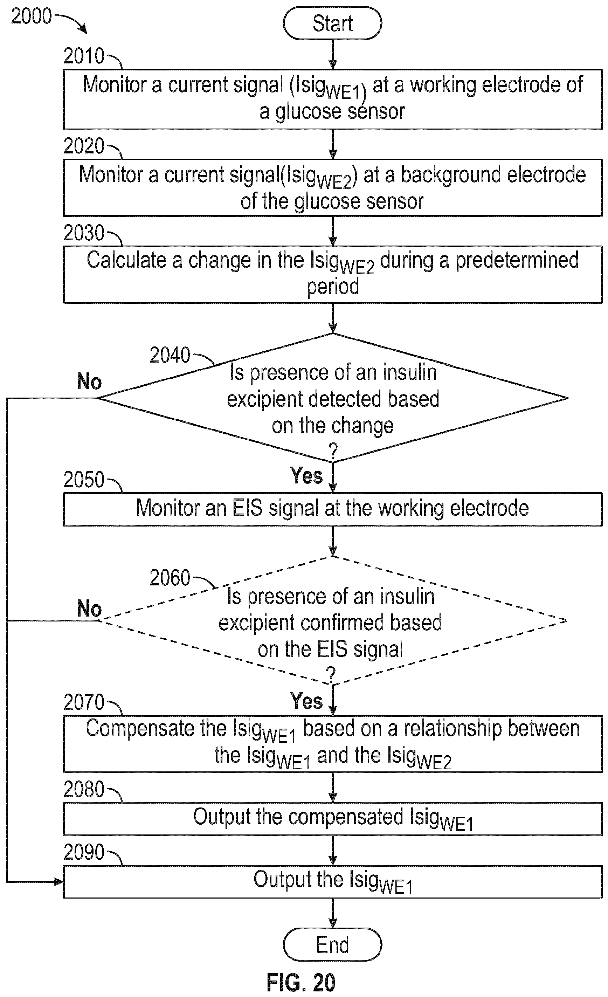

FIG. 20 shows a flowchart of a method 2000 for detecting presence of an excipient in insulin by a glucose sensor in accordance with aspects of the present disclosure. The glucose sensor may include a working electrode, a background electrode, and a controller. The method 2000 starts by monitoring a current signal (Isig_{WE1}) at the working electrode by the controller of the glucose sensor in step 2010 and monitoring a current signal (Isig_{WE2}) at the background electrode by the controller in step 2020. The controller calculates a change in the Isig_{WE2} during the predetermined period is calculated by the controller in step 2030.

In step 2040, it is determined whether or not the change (e.g., an increase or a decrease) during the predetermined period is greater than the predetermined threshold. In a case where the change is determined to be greater than the predetermined threshold, there is presence of excipients of insulin. In a case where the change in the Isig_{WE2} is not greater than the predetermined threshold, it is determined that there is no presence of excipients of insulin.

In a case when it is determined that there is no presence of excipients of insulin, Isig_{WE1}, which is indicative of a level of insulin, is output in step 2090.

In a case when it is determined that there are excipients of insulin, the controller further monitors an EIS signal at the working electrode in step 2050. It is further determined whether or not the presence of excipients of insulin is confirmed based on the EIS signal in step 2060. The confirmation of presence of excipients of insulin may be optional. In an aspect, the confirmation may be also determined based on the EIS signal at the background electrode in step 2050. In another aspect, the EIS signal may be of at least one parameter, which is a real or imaginary impedance at 0.1 Hz.

In a case when the presence of excipients of insulin is not confirmed in step 2060, the Isig_{WE1} is output in step 2090.

In a case when the presence of excipients of insulin is confirmed in step 2060, the Isig_{WE1} is compensated based on a linear relationship between the Isig_{WE1} and Isig_{WE2} in step 2070. As described above with respect to FIG. 9, the compensated Isig_{WE1} (Isig_{COMP}) is equal to: ${Isig}_{WE 1} - α * {Isig}_{WE 2} .$ The Isig_{COMP} is then output in step 2080.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, the above-described servers and computing devices.

Additional steps and changes to the order of the algorithms can be made while still performing the key teachings of the present disclosure. The presently disclosed aspects are to be considered in all respects as illustrative and not restrictive, the scope of the invention being indicated by the appended claims rather than the foregoing description. Unless the context indicates otherwise, any aspect disclosed herein may be combined with any other aspect or aspects disclosed herein.

## Claims

1. A glucose sensor (710) comprising:
a working electrode (534) configured to provide a current signal (Isig_{WE1}) based on a level of glucose;
a background electrode configured to provide a current signal (Isig_{WE2}) based on a presence of an excipient of insulin; and
a controller configured to:
monitor the Isig_{WE1} at the working electrode;
monitor the Isig_{WE2} at the background electrode;
monitor at least one electrochemical impedance spectroscopy (EIS) parameter at the working electrode;
calculate a change in the at least one EIS parameter after an injection of insulin;
detect the presence of the excipient of insulin based on the change; and
in a case where the presence of the excipient of insulin is detected,
compensate the Isig_{WE1} based on a predetermined relationship between the Isig_{WE1} and the Isig_{WE2}; and
output the compensated Isig_{WE1} (Isig_{COMP}).

2. The glucose sensor of claim 1, wherein the at least one EIS parameter is a real impedance at 0.1 Hz,
and/or
wherein the at least one EIS parameter is an imaginary impedance at 0.1 Hz,
and/or
wherein the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

3. The glucose sensor of claim 1 or 2, wherein the predetermined relationship between Isig_{WE1} and the Isig_{WE2} is a linear relationship,
wherein the Isig_{COMP} is equal to: ${Isig}_{WE 1} - α * {Isig}_{WE 2} ,$
where α is a slope of the linear relationship,
wherein the slope has a profile based on a number of injections of insulin,
further comprising:
a memory storing the number of injections of insulin and the profile.

4. A glucose sensor system (700) comprising:
an insulin cannula (730) connected to an insulin pump (740) and configured to inject insulin therethrough; and
the glucose sensor according to claim 1 or 2.

5. The glucose sensor system of claim 4, wherein the predetermined relationship between the Isig_{WE1} and the Isig_{WE2} is a linear relationship,
wherein the Isig_{COMP} is equal to: ${Isig}_{WE 1} - α * {Isig}_{WE 2} ,$
where α is a slope of the linear relationship,
wherein the slope has a profile based on a number of injections of insulin,
wherein the profile is based on a distance between the cannula and the background electrode, further comprising:
a memory storing the number of injections of insulin and the profile.

6. A method for compensating a level of glucose from a glucose sensor (710) based on a presence of an excipient of insulin, the method comprising:
monitoring a current signal (Isig_{WE1}) at a working electrode (534) of the glucose sensor;
monitoring a current signal (Isig_{WE2}) at a background electrode of the glucose sensor;
monitoring at least one electrochemical impedance spectroscopy (EIS) parameter at the working electrode;
calculating a change in the at least one EIS parameter after an injection of insulin;
detecting the presence of the excipient of insulin based on the change; and
in a case where the presence of the excipient of insulin is detected,
compensating the Isig_{WE1} based on a predetermined relationship between the Isig_{WE1} and the Isig_{WE2}; and
outputting the compensated Isig_{WE1} (Isig_{COMP}),
particularly
wherein the at least one EIS parameter is a real impedance at 0.1 Hz, wherein the at least one EIS parameter is an imaginary impedance at 0.1 Hz, wherein the predetermined relationship between the Isig_{WE1} and the Isig_{WE2} is a linear relationship, wherein the Isig_{COMP} is equal to: ${Isig}_{WE 1} - α * {Isig}_{WE 2} ,$
where α is a slope of the linear relationship, wherein the slope has a profile based on a number of injections of insulin, and further comprising: storing the number of injections of insulin and the profile in a memory of the glucose sensor, wherein the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

7. A nontransitory computer-readable medium storing instructions that, when executed by the controller of the glucose sensor (710) of one of claims 1 to 3, cause the controller ·to perform a method for compensating a level of glucose from the glucose sensor (710) based on a presence of an excipient of insulin, the method comprising:
monitoring a current signal (Isig_{WE1}) at a working electrode (534) of the glucose sensor;
monitoring a current signal (Isig_{WE2}) at a background electrode of the glucose sensor;
monitoring at least one electrochemical impedance spectroscopy (EIS) parameter at the working electrode;
calculating a change in the at least one EIS parameter after an injection of insulin;
detecting the presence of the excipient of insulin based on the change; and
in a case where the presence of the excipient of insulin is detected,
compensating the Isig_{WE1} based on a predetermined relationship between the Isig_{WE1} and the Isig_{WE2}; and
outputting the compensated Isig_{WE1} (Isig_{COMP}).

8. A glucose sensor (710) comprising:
a working electrode (534) configured to provide a current signal (Isig_{WE1}) based on a level of glucose;
a background electrode configured to provide a current signal (Isig_{WE2}) based on presence of excipient of insulin;
a controller configured to:
monitor the Isig_{WE1} at the working electrode;
monitor the Isig_{WE2} at the background electrode;
calculate a change in the Isig_{WE2} after an injection of insulin;
detect a presence of an excipient of insulin based on the change; and
in a case where the presence of the excipient of insulin is detected,
compensate the Isig_{WE1} based on a predetermined relationship between the Isig_{WE1} and the Isig_{WE2}; and
output the compensated Isig_{WE1} (Isig_{COMP}).

9. The glucose sensor of claim 8, wherein the predetermined relationship between the Isig_{WE1} and the Isig_{WE2} is a linear relationship,
wherein the Isig_{COMP} is equal to: ${Isig}_{WE 1} - α * {Isig}_{WE 2} ,$
where α is a slope of the linear relationship,
wherein the slope has a profile based on a number of injections of insulin,
further comprising:
a memory storing the number of injections of insulin and the profile.

10. The glucose sensor of claim 8 or 9, wherein a range of voltage applied to the background electrode is from 500 mV to 600 mV, and/or
wherein the controller is further configured to:
monitor an electrochemical impedance spectroscopy (EIS) signal at the working electrode; and
confirm the presence of the excipient of insulin based on the EIS signal of at least one parameter, wherein the at least one parameter is a real impedance at 0.1 Hz of the EIS signal, wherein the at least one parameter is an imaginary impedance at 0.1 Hz of the EIS signal,
and/or
wherein the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

11. A glucose sensor system (700) comprising:
an insulin cannula (730) connected to an insulin pump (740) and configured to inject insulin therethrough;
and the glucose sensor of claim 8.

12. The glucose sensor system of claim 11, wherein the predetermined relationship between the Isig_{WE1} and the Isig_{WE2} is a linear relationship,
wherein the Isig_{COMP} is equal to: ${Isig}_{WE 1} - α * {Isig}_{WE 2} ,$
where α is a slope of the linear relationship,
wherein the slope depends on a distance between the cannula and the working electrode, wherein the slope has a profile based on a number of injections of insulin, wherein the profile is based on a distance between the cannula and the background electrode, further comprising: a memory storing the number of injections of insulin and the profile.

13. The glucose sensor system of claim 11 or 12, wherein a range of voltage applied to the background electrode is from 500 mV to 600 mV, and/or
wherein the controller is further configured to:
monitor at least one electrochemical impedance spectroscopy (EIS) parameter at the working electrode; and
confirm the presence of the excipient of insulin based on the at least one EIS parameter, wherein the at least one EIS parameter is a real impedance at 0.1 Hz of the EIS signal, wherein the at least one EIS parameter is an imaginary impedance at 0.1 Hz of the EIS signal,
and/or wherein the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

14. A method for compensating a level of glucose from a glucose sensor (710) based on presence of an excipient of insulin, the method comprising:
monitoring a current signal (Isig_{WE1}) at a working electrode (534) of the glucose sensor;
monitoring a current signal (Isig_{WE2}) at a background electrode of the glucose sensor;
calculating a change in the Isig_{WE2} after an injection of insulin;
detecting a presence of an excipient of insulin based on the change; and
in a case where the presence of the excipient of insulin is detected,
compensating the Isig_{WE1} based on a predetermined relationship between the Isig_{WE1} and the Isig_{WE2}; and
outputting the compensated Isig_{WE1} (Isig_{COMP}),
particularly wherein the predetermined relationship between the Isig_{WE1} and the Isig_{WE2} is a linear relationship, wherein the Isig_{COMP} is equal to: ${Isig}_{WE 1} - α * {Isig}_{WE 2} ,$ where α is a slope of the linear relationship, wherein the slope depends on a distance between a cannula, through which insulin is injected, and the working electrode, wherein the slope has a profile based on a number of injections of insulin, wherein the more time insulin is injected, the lower the slope is, wherein a range of voltage applied to the background electrode is from 500 mV to 600 mV, further comprising: monitoring at least one electrochemical impedance spectroscopy (EIS) parameter at the working electrode; and confirming the presence of the excipient of insulin based on the at least one EIS parameter, wherein the at least one EIS parameter is a real impedance at 0.1 Hz, wherein the at least one EIS parameter is an imaginary impedance at 0.1 Hz, wherein the excipient of insulin includes one or more of peroxide, phenol, M-cresol, glycerol, zinc, zinc oxide, disodium phosphate, sodium chloride, sodium hydroxide, hydrogen chloride, niacinamide, and arginine hydrochloride.

15. A nontransitory computer-readable medium storing instructions that, when executed by the controller of the glucose sensor (710) of one of claims 8 to 10, cause the controller to perform a method for compensating a level of glucose from the glucose sensor (710) based on a presence of an excipient of insulin, the method comprising:
monitoring a current signal (Isig_{WE1}) at a working electrode (534) of the glucose sensor;
monitoring a current signal (Isig_{WE2}) at a background electrode of the glucose sensor;
calculating a change in the Isig_{WE2} after an injection of insulin;
detecting the presence of the excipient of insulin based on the change; and
in a case where the presence of the excipient of insulin is detected,
compensating the Isig_{WE1} based on a predetermined relationship between the Isig_{WE1} and the Isig_{WE2}; and
outputting the compensated Isig_{WE1} (Isig_{COMP}).

## Patentansprüche

1. Glukosesensor (710), umfassend:
eine Arbeitselektrode (534), die konfiguriert ist, um basierend auf einem Glukosespiegel ein Stromsignal (Isig_{WE1}) bereitzustellen;
eine Hintergrundelektrode, die konfiguriert ist, um basierend auf einem Vorhandensein eines Arzneistoffträgers von Insulin ein Stromsignal (Isig_{WE2}) bereitzustellen; und
eine Steuerung, die konfiguriert ist zum:
Überwachen des Isig_{WE1} an der Arbeitselektrode;
Überwachen des Isig_{WE2} an der Hintergrundelektrode;
Überwachen mindestens eines Parameters einer elektrochemischen Impedanzspektroskopie (EIS) an der Arbeitselektrode;
Berechnen einer Änderung des mindestens einen EIS-Parameters nach einer Injektion von Insulin;
Nachweisen des Vorhandenseins des Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}).

2. Glukosesensor nach Anspruch 1, wobei der mindestens eine EIS-Parameter eine reale Impedanz bei 0,1 Hz ist,
und/oder
wobei der mindestens eine EIS-Parameter eine imaginäre Impedanz bei 0,1 Hz ist,
und/oder
wobei der Arzneistoffträger von Insulin eines oder mehrere von Peroxid, Phenol, M-Kresol, Glycerol, Zink, Zinkoxid, Dinatriumphosphat, Natriumchlorid, Natriumhydroxid, Chlorwasserstoff, Niacinamid und Argininhydrochlorid einschließt.

3. Glukosesensor nach Anspruch 1 oder 2, wobei die vorbestimmte Beziehung zwischen Isig_{WE1} und dem Isig_{WE2} eine lineare Beziehung ist,
wobei das Isig_{COMP} gleich ist: ${Isig}_{WE 1} - α * {Isig}_{WE 2} ,$
wobei α eine Steigung der linearen Beziehung ist,
wobei die Steigung ein Profil basierend auf einer Anzahl von Injektionen von Insulin aufweist,
ferner umfassend:
einen Speicher, der die Anzahl der Injektionen von Insulin und das Profil speichert.

4. Glukosesensorsystem (700), umfassend:
eine Insulinkanüle (730), die mit einer Insulinpumpe (740) verbunden und konfiguriert ist, um Insulin durch diese hindurch zu injizieren; und
den Glucosesensor nach Anspruch 1 oder 2.

5. Glukosesensorsystem nach Anspruch 4, wobei die vorbestimmte Beziehung zwischen dem Isig_{WE1} und dem Isig_{WE2} eine lineare Beziehung ist,
wobei das Isig_{COMP} gleich ist: ${Isig}_{WE 1} - α * {Isig}_{WE 2} ,$
wobei α eine Steigung der linearen Beziehung ist,
wobei die Steigung ein Profil basierend auf einer Anzahl von Injektionen von Insulin aufweist,
wobei das Profil auf einem Abstand zwischen der Kanüle und der Hintergrundelektrode basiert, ferner umfassend:
einen Speicher, der die Anzahl der Injektionen von Insulin und das Profil speichert.

6. Verfahren zum Ausgleichen eines Glukosespiegels von einem Glukosesensor (710) basierend auf einem Vorhandensein eines Arzneistoffträgers von Insulin, das Verfahren umfassend:
Überwachen eines Stromsignals (Isig_{WE1}) an einer Arbeitselektrode (534) des Glukosesensors;
Überwachen eines Stromsignals (Isig_{WE2}) an einer Hintergrundelektrode des Glukosesensors;
Überwachen mindestens eines Parameters der elektrochemischen Impedanzspektroskopie (EIS) an der Arbeitselektrode;
Berechnen einer Änderung des mindestens einen EIS-Parameters nach einer Injektion von Insulin;
Nachweisen des Vorhandenseins des Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}),
insbesondere
wobei der mindestens eine EIS-Parameter eine reale Impedanz bei 0,1 Hz ist, wobei der mindestens eine EIS-Parameter eine imaginäre Impedanz bei 0,1 Hz ist, wobei die vorbestimmte Beziehung zwischen dem Isig_{WE1} und dem Isig_{WE2} eine lineare Beziehung ist, wobei das Isig_{COMP} gleich ist: ${Isig}_{WE 1} - α * {Isig}_{WE 2} ,$
wobei α eine Steigung der linearen Beziehung ist, wobei die Steigung ein Profil basierend auf einer Anzahl von Injektionen von Insulin aufweist, und ferner umfassend: Speichern der Anzahl von Injektionen von Insulin und des Profils in einem Speicher des Glukosesensors, wobei der Arzneistoffträger von Insulin eines oder mehrere von Peroxid, Phenol, M-Kresol, Glycerol, Zink, Zinkoxid, Dinatriumphosphat, Natriumchlorid, Natriumhydroxid, Chlorwasserstoff, Niacinamid und Argininhydrochlorid einschließt.

7. Nichtflüchtiges computerlesbares Medium, das Anweisungen speichert, die, wenn sie durch die Steuerung des Glukosesensors (710) nach einem der Ansprüche 1 bis 3 ausgeführt werden,
die Steuerung veranlassen, ein Verfahren zum Ausgleichen eines Glukosespiegels von dem Glukosesensor (710) basierend auf einem Vorhandensein eines Arzneistoffträgers von Insulin durchzuführen, das Verfahren umfassend:
Überwachen eines Stromsignals (Isig_{WE1}) an einer Arbeitselektrode (534) des Glukosesensors;
Überwachen eines Stromsignals (Isig_{WE2}) an einer Hintergrundelektrode des Glukosesensors;
Überwachen mindestens eines Parameters der elektrochemischen Impedanzspektroskopie (EIS) an der Arbeitselektrode;
Berechnen einer Änderung des mindestens einen EIS-Parameters nach einer Injektion von Insulin;
Nachweisen des Vorhandenseins des Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}).

8. Glukosesensor (710), umfassend:
eine Arbeitselektrode (534), die konfiguriert ist, um basierend auf einem Glukosespiegel ein Stromsignal (Isig_{WE1}) bereitzustellen;
eine Hintergrundelektrode, die konfiguriert ist, um basierend auf dem Vorhandensein eines Arzneistoffträgers von Insulin ein Stromsignal (Isig_{WE2}) bereitzustellen;
eine Steuerung, die konfiguriert ist zum:
Überwachen des Isig_{WE1} an der Arbeitselektrode;
Überwachen des Isig_{WE2} an der Hintergrundelektrode;
Berechnen einer Änderung des Isig_{WE2} nach einer Injektion von Insulin;
Nachweisen eines Vorhandenseins eines Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}).

9. Glukosesensor nach Anspruch 8, wobei die vorbestimmte Beziehung zwischen dem Isig_{WE1} und dem Isig_{WE2} eine lineare Beziehung ist,
wobei das Isig_{COMP} gleich ist: ${Isig}_{WE 1} - α * {Isig}_{WE 2} ,$
wobei α eine Steigung der linearen Beziehung ist,
wobei die Steigung ein Profil basierend auf einer Anzahl von Injektionen von Insulin aufweist,
ferner umfassend:
einen Speicher, der die Anzahl der Injektionen von Insulin und das Profil speichert.

10. Glukosesensor nach Anspruch 8 oder 9, wobei ein Bereich einer Spannung, die an die Hintergrundelektrode angelegt ist, von 500 mV bis 600 mV reicht, und/oder
wobei die Steuerung ferner konfiguriert ist zum:
Überwachen eines Signals der elektrochemischen Impedanzspektroskopie (EIS) an der Arbeitselektrode; und
Bestätigen des Vorhandenseins des Arzneistoffträgers von Insulin basierend auf dem EIS-Signal von mindestens einem Parameter, wobei der mindestens eine Parameter eine reale Impedanz bei 0,1 Hz des EIS-Signals ist, wobei der mindestens eine Parameter eine imaginäre Impedanz bei 0,1 Hz des EIS-Signals ist, und/oder
wobei der Arzneistoffträger von Insulin eines oder mehrere von Peroxid, Phenol, M-Kresol, Glycerol, Zink, Zinkoxid, Dinatriumphosphat, Natriumchlorid, Natriumhydroxid, Chlorwasserstoff, Niacinamid und Argininhydrochlorid einschließt.

11. Glukosesensorsystem (700), umfassend:
eine Insulinkanüle (730), die mit einer Insulinpumpe (740) verbunden und konfiguriert ist, um Insulin durch diese hindurch zu injizieren;
und den Glukosesensor nach Anspruch 8.

12. Glukosesensorsystem nach Anspruch 11, wobei die vorbestimmte Beziehung zwischen dem Isig_{WE1} und dem Isig_{WE2} eine lineare Beziehung ist,
wobei das Isig_{COMP} gleich ist: ${Isig}_{WE 1} - α * {Isig}_{WE 2} ,$
wobei α eine Steigung der linearen Beziehung ist,
wobei die Steigung von einem Abstand zwischen der Kanüle und der Arbeitselektrode abhängt, wobei die Steigung ein Profil aufweist, das auf einer Anzahl von Injektionen von Insulin basiert, wobei das Profil auf einem Abstand zwischen der Kanüle und der Hintergrundelektrode basiert, ferner umfassend: einen Speicher, der die Anzahl von Injektionen von Insulin und das Profil speichert.

13. Glukosesensorsystem nach Anspruch 11 oder 12, wobei ein Bereich der Spannung, die an die Hintergrundelektrode angelegt ist, von 500 mV bis 600 mV reicht, und/oder wobei die Steuerung ferner konfiguriert ist zum:
Überwachen mindestens eines Parameters der elektrochemischen Impedanzspektroskopie (EIS) an der Arbeitselektrode; und
Bestätigen des Vorhandenseins des Arzneistoffträgers von Insulin basierend auf dem mindestens einen EIS-Parameter, wobei der mindestens eine EIS-Parameter eine reale Impedanz bei 0,1 Hz des EIS-Signals ist, wobei der mindestens eine EIS-Parameter eine imaginäre Impedanz bei 0,1 Hz des EIS-Signals ist,
und/oder wobei der Arzneistoffträger von Insulin eines oder mehrere von Peroxid, Phenol, M-Kresol, Glycerol, Zink, Zinkoxid, Dinatriumphosphat, Natriumchlorid, Natriumhydroxid, Chlorwasserstoff, Niacinamid und Argininhydrochlorid einschließt.

14. Verfahren zum Ausgleichen eines Glukosespiegels von einem Glukosesensor (710) basierend auf dem Vorhandensein eines Arzneistoffträgers von Insulin, das Verfahren umfassend:
Überwachen eines Stromsignals (Isig_{WE1}) an einer Arbeitselektrode (534) des Glukosesensors;
Überwachen eines Stromsignals (Isig_{WE2}) an einer Hintergrundelektrode des Glukosesensors;
Berechnen einer Änderung des Isig_{WE2} nach einer Injektion von Insulin;
Nachweisen eines Vorhandenseins eines Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}),
insbesondere wobei die vorbestimmte Beziehung zwischen dem Isig_{WE1} und dem Isig_{WE2} eine lineare Beziehung ist, wobei das Isig_{COMP} gleich ist: ${Isig}_{WE 1} - α * {Isig}_{WE 2} ,$
wobei α eine Steigung der linearen Beziehung ist, wobei die Steigung von einem Abstand zwischen einer Kanüle, durch die Insulin injiziert wird, und der Arbeitselektrode abhängt, wobei die Steigung ein Profil aufweist, das auf einer Anzahl von Injektionen von Insulin basiert, wobei, je häufiger Insulin injiziert wird, desto geringer die Steigung ist, wobei ein Bereich der Spannung, die an die Hintergrundelektrode angelegt ist, von 500 mV bis 600 mV reicht, ferner umfassend: Überwachen mindestens eines Parameters der elektrochemischen Impedanzspektroskopie (EIS) an der Arbeitselektrode; und Bestätigen des Vorhandenseins des Arzneistoffträgers von Insulin basierend auf dem mindestens einen EIS-Parameter, wobei der mindestens eine EIS-Parameter eine reale Impedanz bei 0,1 Hz ist, wobei der mindestens eine EIS-Parameter eine imaginäre Impedanz bei 0,1 Hz ist, wobei der Arzneistoffträger von Insulin eines oder mehrere von Peroxid, Phenol, M-Kresol, Glycerin, Zink, Zinkoxid, Dinatriumphosphat, Natriumchlorid, Natriumhydroxid, Chlorwasserstoff, Niacinamid und Argininhydrochlorid einschließt.

15. Nichtflüchtiges computerlesbares Medium, das Anweisungen speichert, die, wenn sie durch die Steuerung des Glukosesensors (710) nach einem der Ansprüche 8 bis 10 ausgeführt werden,
die Steuerung veranlassen, ein Verfahren zum Ausgleichen eines Glukosespiegels von dem Glukosesensor (710) basierend auf einem Vorhandensein eines Arzneistoffträgers von Insulin durchzuführen, das Verfahren umfassend:
Überwachen eines Stromsignals (Isig_{WE1}) an einer Arbeitselektrode (534) des Glukosesensors;
Überwachen eines Stromsignals (Isig_{WE2}) an einer Hintergrundelektrode des Glukosesensors;
Berechnen einer Änderung des Isig_{WE2} nach einer Injektion von Insulin;
Nachweisen des Vorhandenseins des Arzneistoffträgers von Insulin basierend auf der Änderung; und
in einem Fall, in dem das Vorhandensein des Arzneistoffträgers von Insulin nachgewiesen wird,
Ausgleichen des Isig_{WE1} basierend auf einer vorbestimmten Beziehung zwischen dem Isig_{WE1} und dem Isig_{WE2}; und
Ausgeben des ausgeglichenen Isig_{WE1} (Isig_{COMP}).

## Revendications

1. Capteur de glucose (710) comprenant :
une électrode de travail (534) configurée pour fournir un signal de courant (Isig_{WE1}) en fonction d'un taux de glucose ;
une électrode de fond configurée pour fournir un signal de courant (Isig_{WE2}) en fonction d'une présence d'un excipient d'insuline ; et
une unité de commande configurée pour :
surveiller l'Isig_{WE1} au niveau de l'électrode de travail ;
surveiller l'Isig_{WE2} au niveau de l'électrode de fond ;
surveiller au moins un paramètre de spectroscopie d'impédance électrochimique (EIS) au niveau de l'électrode de travail ;
calculer un changement dans l'au moins un paramètre EIS après une injection d'insuline ;
détecter la présence de l'excipient d'insuline en fonction du changement ; et
dans un cas où la présence de l'excipient d'insuline est détectée,
compenser l'Isig_{WE1} en fonction d'une relation prédéterminée entre l'Isig_{WE1} et l'Isig_{WE2} ; et
délivrer en sortie l'Isig_{WE1} compensé (Isig_{COMP}).

2. Capteur de glucose selon la revendication 1, dans lequel l'au moins un paramètre EIS est une impédance réelle à 0,1 Hz,
et/ou
dans lequel l'au moins un paramètre EIS est une impédance imaginaire à 0,1 Hz,
et/ou
dans lequel l'excipient d'insuline comporte un ou plusieurs parmi peroxyde, phénol, M-crésol, glycérol, zinc, oxyde de zinc, phosphate disodique, chlorure de sodium, hydroxyde de sodium, chlorure d'hydrogène, niacinamide et chlorhydrate d'arginine.

3. Capteur de glucose selon la revendication 1 ou 2, dans lequel la relation prédéterminée entre Isig_{WE1} et l'Isig_{WE2} est une relation linéaire,
dans lequel l'Isig_{COMP} est égal à : ${Isig}_{WE 1} - α * {Isig}_{WE 2} ,$
où α est une pente de la relation linéaire,
dans lequel la pente a un profil en fonction d'un nombre d'injections d'insuline,
comprenant en outre :
une mémoire stockant le nombre d'injections d'insuline et le profil.

4. Système de capteur de glucose (700) comprenant :
une canule d'insuline (730) raccordée à une pompe à insuline (740) et conçue pour injecter de l'insuline à travers celle-ci ; et
le capteur de glucose selon la revendication 1 ou 2.

5. Système de capteur de glucose selon la revendication 4, dans lequel la relation prédéterminée entre l'Isig_{WE1} et l'Isig_{WE2} est une relation linéaire,
dans lequel l'Isig_{COMP} est égal à : ${Isig}_{WE 1} - α * {Isig}_{WE 2} ,$
où α est une pente de la relation linéaire,
dans lequel la pente a un profil en fonction d'un nombre d'injections d'insuline,
dans lequel le profil est en fonction d'une distance entre la canule et l'électrode de fond, comprenant en outre :
une mémoire stockant le nombre d'injections d'insuline et le profil.

6. Procédé permettant de compenser un taux de glucose provenant d'un capteur de glucose (710) en fonction d'une présence d'un excipient d'insuline, le procédé comprenant :
la surveillance d'un signal de courant (Isig_{WE1}) au niveau d'une électrode de travail (534) du capteur de glucose ;
la surveillance d'un signal de courant (Isig_{WE2}) au niveau d'une électrode de fond du capteur de glucose ;
la surveillance d'au moins un paramètre de spectroscopie d'impédance électrochimique (EIS) au niveau de l'électrode de travail ;
le calcul d'un changement dans l'au moins un paramètre EIS après une injection d'insuline ;
la détection de la présence de l'excipient d'insuline en fonction du changement ; et
dans un cas où la présence de l'excipient d'insuline est détectée,
la compensation de l'Isig_{WE1} en fonction d'une relation prédéterminée entre l'Isig_{WE1} et l'Isig_{WE2} ; et
la sortie de l'Isig_{WE1} compensé (Isig_{COMP}),
en particulier
dans lequel l'au moins un paramètre EIS est une impédance réelle à 0,1 Hz, dans lequel l'au moins un paramètre EIS est une impédance imaginaire à 0,1 Hz, dans lequel la relation prédéterminée entre l'Isig_{WE1} et l'Isig_{WE2} est une relation linéaire, dans lequel l'Isig_{COMP} est égal à : ${Isig}_{WE 1} - α * {Isig}_{WE 2} ,$
où α est une pente de la relation linéaire, dans lequel la pente a un profil en fonction d'un nombre d'injections d'insuline, et comprenant en outre : le stockage du nombre d'injections d'insuline et du profil dans une mémoire du capteur de glucose, dans lequel l'excipient d'insuline comporte un ou plusieurs parmi peroxyde, phénol, M-crésol, glycérol, zinc, oxyde de zinc, phosphate disodique, chlorure de sodium, hydroxyde de sodium, chlorure d'hydrogène, niacinamide et chlorhydrate d'arginine.

7. Support non transitoire lisible par ordinateur stockant des instructions qui, lorsqu'elles sont exécutées par l'unité de commande du capteur de glucose (710) selon l'une des revendications 1 à 3,
amènent l'unité de commande à mettre en œuvre un procédé permettant de compenser un taux de glucose provenant du capteur de glucose (710) en fonction d'une présence d'un excipient d'insuline, le procédé comprenant :
la surveillance d'un signal de courant (Isig_{WE1}) au niveau d'une électrode de travail (534) du capteur de glucose ;
la surveillance d'un signal de courant (Isig_{WE2}) au niveau d'une électrode de fond du capteur de glucose ;
la surveillance d'au moins un paramètre de spectroscopie d'impédance électrochimique (EIS) au niveau de l'électrode de travail ;
le calcul d'un changement dans l'au moins un paramètre EIS après une injection d'insuline ;
la détection de la présence de l'excipient d'insuline en fonction du changement ; et
dans un cas où la présence de l'excipient d'insuline est détectée,
la compensation de l'Isig_{WE1} en fonction d'une relation prédéterminée entre l'Isig_{WE1} et l'Isig_{WE2} ; et
la sortie de l'Isig_{WE1} compensé (Isig_{COMP}).

8. Capteur de glucose (710) comprenant :
une électrode de travail (534) configurée pour fournir un signal de courant (Isig_{WE1}) en fonction d'un taux de glucose ;
une électrode de fond configurée pour fournir un signal de courant (Isig_{WE2}) en fonction d'une présence d'excipient d'insuline ;
une unité de commande configurée pour :
surveiller l'Isig_{WE1} au niveau de l'électrode de travail ;
surveiller l'Isig_{WE2} au niveau de l'électrode de fond ;
calculer un changement dans l'Isig_{WE2} après une injection d'insuline ;
détecter une présence d'un excipient d'insuline en fonction du changement ; et
dans un cas où la présence de l'excipient d'insuline est détectée,
compenser l'Isig_{WE1} en fonction d'une relation prédéterminée entre l'Isig_{WE1} et l'Isig_{WE2} ; et
délivrer en sortie l'Isig_{WE1} compensé (Isig_{COMP}).

9. Capteur de glucose selon la revendication 8, dans lequel la relation prédéterminée entre l'Isig_{WE1} et l'Isig_{WE2} est une relation linéaire,
dans lequel l'Isig_{COMP} est égal à : ${Isig}_{WE 1} - α * {Isig}_{WE 2} ,$
où α est une pente de la relation linéaire,
dans lequel la pente a un profil en fonction d'un nombre d'injections d'insuline,
comprenant en outre :
une mémoire stockant le nombre d'injections d'insuline et le profil.

10. Capteur de glucose selon la revendication 8 ou 9, dans lequel une plage de tension appliquée à l'électrode de fond va de 500 mV à 600 mV, et/ou
dans lequel l'unité de commande est configurée en outre pour :
surveiller un signal de spectroscopie d'impédance électrochimique (EIS) au niveau de l'électrode de travail ; et
confirmer la présence de l'excipient d'insuline en fonction du signal EIS d'au moins un paramètre, dans lequel l'au moins un paramètre est une impédance réelle à 0,1 Hz du signal EIS, dans lequel l'au moins un paramètre est une impédance imaginaire à 0,1 Hz du signal EIS, et/ou
dans lequel l'excipient d'insuline comporte un ou plusieurs parmi peroxyde, phénol, M-crésol, glycérol, zinc, oxyde de zinc, phosphate disodique, chlorure de sodium, hydroxyde de sodium, chlorure d'hydrogène, niacinamide et chlorhydrate d'arginine.

11. Système de capteur de glucose (700) comprenant :
une canule d'insuline (730) raccordée à une pompe à insuline (740) et conçue pour injecter de l'insuline à travers celle-ci ;
et le capteur de glucose selon la revendication 8.

12. Système de capteur de glucose selon la revendication 11, dans lequel la relation prédéterminée entre l'Isig_{WE1} et l'Isig_{WE2} est une relation linéaire,
dans lequel l'Isig_{COMP} est égal à : ${Isig}_{WE 1} - α * {Isig}_{WE 2} ,$
où α est une pente de la relation linéaire,
dans lequel la pente dépend d'une distance entre la canule et l'électrode de travail, dans lequel la pente a un profil en fonction d'un nombre d'injections d'insuline, dans lequel le profil est en fonction d'une distance entre la canule et l'électrode de fond, comprenant en outre : une mémoire stockant le nombre d'injections d'insuline et le profil.

13. Système de capteur de glucose selon la revendication 11 ou 12, dans lequel une plage de tension appliquée à l'électrode de fond va de 500 mV à 600 mV, et/ou dans lequel l'unité de commande est configurée en outre pour :
surveiller au moins un paramètre de spectroscopie d'impédance électrochimique (EIS) au niveau de l'électrode de travail ; et
confirmer la présence de l'excipient d'insuline en fonction de l'au moins un paramètre EIS, dans lequel l'au moins un paramètre EIS est une impédance réelle à 0,1 Hz du signal EIS, dans lequel l'au moins un paramètre EIS est une impédance imaginaire à 0,1 Hz du signal EIS,
et/ou dans lequel l'excipient d'insuline comporte un ou plusieurs parmi peroxyde, phénol, M-crésol, glycérol, zinc, oxyde de zinc, phosphate disodique, chlorure de sodium, hydroxyde de sodium, chlorure d'hydrogène, niacinamide et chlorhydrate d'arginine.

14. Procédé permettant de compenser un taux de glucose provenant d'un capteur de glucose (710) en fonction de la présence d'un excipient d'insuline, le procédé comprenant :
la surveillance d'un signal de courant (Isig_{WE1}) au niveau d'une électrode de travail (534) du capteur de glucose ;
la surveillance d'un signal de courant (Isig_{WE2}) au niveau d'une électrode de fond du capteur de glucose ;
le calcul d'un changement dans l'Isig_{WE2} après une injection d'insuline ;
la détection d'une présence d'un excipient d'insuline en fonction du changement ; et
dans un cas où la présence de l'excipient d'insuline est détectée,
la compensation de l'Isig_{WE1} en fonction d'une relation prédéterminée entre l'Isig_{WE1} et l'Isig_{WE2} ; et
la sortie de l'Isig_{WE1} compensé (Isig_{COMP}),
en particulier dans lequel la relation prédéterminée entre l'Isig_{WE1} et l'Isig_{WE2} est une relation linéaire, dans lequel l'Isig_{COMP} est égal à : ${Isig}_{WE 1} - α * {Isig}_{WE 2} ,$
où α est une pente de la relation linéaire, dans lequel la pente dépend d'une distance entre une canule, à travers laquelle de l'insuline est injectée, et l'électrode de travail, dans lequel la pente a un profil en fonction d'un nombre d'injections d'insuline, dans lequel plus la durée d'injection d'insuline est longue, plus la pente est faible, dans lequel une plage de tension appliquée à l'électrode de fond va de 500 mV à 600 mV, comprenant en outre : la surveillance d'au moins un paramètre de spectroscopie d'impédance électrochimique (EIS) au niveau de l'électrode de travail ; et la confirmation de la présence de l'excipient d'insuline en fonction de l'au moins un paramètre EIS, dans lequel l'au moins un paramètre EIS est une impédance réelle à 0,1 Hz, dans lequel l'au moins un paramètre EIS est une impédance imaginaire à 0,1 Hz, dans lequel l'excipient d'insuline comporte un ou plusieurs parmi peroxyde, phénol, M-crésol, glycérol, zinc, oxyde de zinc, phosphate disodique, chlorure de sodium, hydroxyde de sodium, chlorure d'hydrogène, niacinamide et chlorhydrate d'arginine.

15. Support non transitoire lisible par ordinateur stockant des instructions qui, lorsqu'elles sont exécutées par l'unité de commande du capteur de glucose (710) selon l'une des revendications 8 à 10,
amènent l'unité de commande à mettre en œuvre un procédé permettant de compenser un taux de glucose provenant du capteur de glucose (710) en fonction d'une présence d'un excipient d'insuline, le procédé comprenant :
la surveillance d'un signal de courant (Isig_{WE1}) au niveau d'une électrode de travail (534) du capteur de glucose ;
la surveillance d'un signal de courant (Isig_{WE2}) au niveau d'une électrode de fond du capteur de glucose ;
le calcul d'un changement dans l'Isig_{WE2} après une injection d'insuline ;
la détection de la présence de l'excipient d'insuline en fonction du changement ; et
dans un cas où la présence de l'excipient d'insuline est détectée,
la compensation de l'Isig_{WE1} en fonction d'une relation prédéterminée entre l'Isig_{WE1} et l'Isig_{WE2} ; et
la sortie de l'Isig_{WE1} compensé (Isig_{COMP}).
